(19) Europäisches Patentamt
European Patent Office
Office européen des brevets

(11) **EP 2 626 058 B1**

(12) **EUROPEAN PATENT SPECIFICATION**

(45) Date of publication and mention
of the grant of the patent:
**01.05.2019 Bulletin 2019/18**

(51) Int Cl.:
*A61K 6/00* (2006.01)  *A61K 8/24* (2006.01)
*A61Q 11/00* (2006.01)  *A61K 6/06* (2006.01)
*A61K 8/19* (2006.01)  *A61K 6/033* (2006.01)

(21) Application number: **11830599.4**

(22) Date of filing: **30.09.2011**

(86) International application number:
**PCT/JP2011/072684**

(87) International publication number:
**WO 2012/046667 (12.04.2012 Gazette 2012/15)**

(54) **DENTINAL TUBULE SEALANT AND METHOD FOR PRODUCING THE SAME**

MITTEL ZUR VERSIEGELUNG DER ZAHN-CANALICULI UND VERFAHREN ZU SEINER HERSTELLUNG

AGENT OBTURANT LES CANAUX DENTAIRES ET PROCÉDÉ DE PRODUCTION CORRESPONDANT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.10.2010 JP 2010227058**

(43) Date of publication of application:
**14.08.2013 Bulletin 2013/33**

(73) Proprietor: **KURARAY NORITAKE DENTAL INC.**
**Kurashiki-shi**
**Okayama**
**710-0801 (JP)**

(72) Inventors:
• **HASHIMOTO, Tadashi**
**Tokyo 100-8115 (JP)**
• **OSHITA, Mizuko**
**Kurashiki-shi**
**Okayama 710-0801 (JP)**

• **ISHIHARA, Shumei**
**Kurashiki-shi**
**Okayama 710-0801 (JP)**

(74) Representative: **Müller-Boré & Partner**
**Patentanwälte PartG mbB**
**Friedenheimer Brücke 21**
**80639 München (DE)**

(56) References cited:
EP-A1- 0 639 366    JP-A- 1 022 257
JP-A- 3 151 313    JP-A- H10 504 467
US-A- 5 782 971    US-A- 5 997 624
US-A- 6 053 970    US-A1- 2001 046 475

Remarks:
The file contains technical information submitted after the application was filed and not included in this specification

**Description**

TECHNICAL FIELD

The present invention relates to a dentinal tubule sealant capable of sealing dentinal tubules of an exposed dentin.

BACKGROUND ART

Along with a so-called "8020 campaign" (improvement in dental health, preservation of dentin (MI: Minimal Intervention)) to try to keep 20 or more own teeth even when being 80 years old, a mineralization therapy in which mineralization is performed at an initial caries stage before affection to caries, thereby returning the caries to healthy tooth substance has recently got into the spotlight. From this point of view, functional gums, dentifrices, and tooth surface-treating materials in which fluorine or a calcium solubilizing agent (CPP-ACP; Casein PhosphoPeptide-Amorphous Calcium Phosphate, POs-Ca (registered trademark); phosphoryl oligosaccharides of calcium) is included as an active ingredient have been sold by various companies. However, fluorine has been believed to have a function to improve the acid resistance of teeth, thereby strengthening minerals of tooth substance, but it has a problem of side effects caused by its intake in a large amount. Moreover, there has also been a problem that a material including a calcium solubilizing agent is low in ability to deposit minerals due to its high solubility though the material can supply a high concentration of minerals to the vicinity of tooth substance.

Patent Document 1 discloses a curable composition having, as basic components, a malaxation liquid and a mixture of at least one member selected from the group consisting of dicalcium phosphate, tricalcium phosphate, octacalcium phosphate, and calcium dihydrogen phosphate and at least one member selected from the group consisting of tetracalcium phosphate, calcium oxide, and calcium hydroxide, wherein the malaxation liquid at the time of malaxation has a phosphate ion concentration of 30 mmol concentration or more or a pH of 3 or less or a pH of 10 or more. This has reported that there can be provided a curable composition being excellent in biocompatibility, having formability, and having a curing time of up to 20 minutes, which can be used for clinical applications. Moreover, this curable composition has been reported to adapt itself to a lost part and a cavity of a hard tissue, such as a bone and a tooth, form a calcium phosphate cured body of a desired form at these sites, make up for the function of lost cavities, and induce the generation of a new hard tissue.

Patent Document 2 discloses a method for making a calcium phosphate cement which self-sets to hydroxyapatite as a major product at ambient temperatures, the method comprising combining a calcium phosphate salt having a calcium to phosphorus molar ratio of less than 5/3 which is other than tetracalcium phosphate with an additional source of calcium and an aqueous solution adjusted with base to maintain a pH of about 12.5 or above and having a concentration of phosphate of about 0.2 mol/L or above, in the absence of solid crystalline phosphoric acid. This self-setting calcium phosphate has been reported to have a merit that it sets particularly rapidly and be expected to be used as a prosthesis that repairs dental and surgery deficits.

Patent Document 3 discloses a liquid product for remineralizing subsurface lesions and for mineralizing exposed dentin tubules in teeth, the product comprising a mixed aqueous composition of a cationic component comprising at least one partially water-soluble calcium salt, an anionic component comprising a water-soluble phosphoric acid salt and a water-soluble fluoride salt, and water. As used herein, the partially water-soluble calcium salt is a calcium salt having a solubility greater than that of dicalcium phosphate dihydrate (DCPD) in an aqueous solution having a pH of 7.0 and a temperature of 25°C, which is defined to have a solubility capable of releasing more than 40 ppm and not more than 1400 ppm of calcium cations. Specifically, calcium sulfate, anhydrous calcium sulfate, calcium sulfate hemihydrate, calcium sulfate dihydrate, calcium malate, calcium tartrate, calcium malonate and calcium succinate have been provided as examples. Moreover, it is disclosed that an abrasive agent may be contained in a toothpaste, a gel, and a cream product. Specific examples disclosed of the abrasive agent include β-phase calcium pyrophosphate, dicalcium phosphate dihydrate, anhydrous calcium phosphate, calcium carbonate, zirconium silicate, and thermosetting resins.

Patent Document 4 discloses storage stable dental cements comprising poorly soluble calcium phosphate particles, calcium compounds and water.

As disclosed in the above-cited prior patent documents, a dentin remineralization agent comprising a water-soluble calcium salt and a water-soluble phosphoric acid salt as main components or a dentin remineralization agent comprising a poorly-soluble calcium phosphate, a large amount of water-soluble calcium salt, and a water-soluble phosphoric acid salt as main components has been used in the conventional technologies. It has been reported that these components dissolve in an aqueous solution to generate a calcium ion and a phosphate ion, which deposit as hydroxyapatite (this may hereinafter be abbreviated as HAp) to remineralize dentin or dentinal tubules opening in an exposed dentin. However, there was no disclosure that a dentinal tubule sealant with a high dentinal tubule inhibition ratio can be obtained by inclusion of poorly-soluble calcium phosphate particles, a phosphorus-free calcium compound, and water in certain amounts . Dentinal tubules opening in an exposed dentin have a diameter of about 2 $\mu$m, and in order to seal dentinal tubules completely with deposited HAp, a long time or repeated application of a material is needed, so that there are problems in practicality.

PRIOR ART DOCUMENTS

PATENT DOCUMENTS

Patent Document 1: JP 6-172008 A
Patent Document 2: JP 10-504467 A
Patent Document 3: JP 2000-504037 A
Patent Document 4: US 6 053 970

DISCLOSURE OF THE INVENTION

PROBLEMS TO BE SOLVED BY THE INVENTION

The present invention was devised in order to solve the above-described problems, and an object thereof is to provide a dentinal tubule sealant which is capable of sealing dentinal tubules of an exposed dentin and remineralizing the surrounding dentin after the sealing.

MEANS FOR SOLVING THE PROBLEMS

The above-described problems can be solved by providing a dentinal tubule sealant comprising poorly-soluble calcium phosphate particles (A), a phosphorus-free calcium compound (B), and water (C), wherein the poorly-soluble calcium phosphate particles (A) are at least one member selected from the group consisting of dicalcium phosphate anhydrous [$CaHPO_4$] particles, $\alpha$-tricalcium phosphate [$\alpha$-$Ca_3(PO_4)_2$] particles, $\beta$-tricalcium phosphate [$\beta$-$Ca_3(PO_4)_2$] particles, amorphous calcium phosphate [$Ca_3(PO_4)_2 \cdot nH_2O$] particles, calcium pyrophosphate [$Ca_2P_2O_7$] particles, calcium pyrophosphate dihydrate [$Ca_2P_2O_7 \cdot 2H_2O$] particles, octacalcium phosphate pentahydrate [$Ca_8H_2(PO_4)_6 \cdot 5H_2O$] particles, and dicalcium phosphate dihydrate [$CaHPO_4 \cdot 2H_2O$] particles, and the dentinal tubule sealant contains 30 to 76% by weight of the poorly-soluble calcium phosphate particles (A), 0.01 to 2% by weight of the phosphorus-free calcium compound (B), and 23 to 69% by weight of the water (C).

In this embodiment, it is preferred that the phosphorus-free calcium compound (B) is at least one member selected from the group consisting of calcium hydroxide [$Ca(OH)_2$], calcium oxide [$CaO$], calcium chloride [$CaCl_2$], calcium nitrate [$Ca(NO_3)_2 \cdot nH_2O$], calcium acetate [$Ca(CH_3CO_2)_2 \cdot nH_2O$], calcium lactate [$C_6H_{10}CaO_6$], calcium citrate [$Ca_3(C_6H_5O_7)_2 \cdot nH_2O$] , calcium metasilicate [$CaSiO_3$], dicalcium silicate [$Ca_2SiO_4$] , tricalcium silicate [$Ca_3SiO_5$] , and calcium carbonate [$CaCO_3$]. It is preferable that the dentinal tubule sealant contains 0.1 to 25% by weight of an alkali metal salt of phosphoric acid (D), and it is also preferable that a Ca/P ratio of the poorly-soluble calcium phosphate particle (A) and the phosphorus-free calcium compound (B) in total is from 0.9 to 1.25. It is preferable that the dentinal tubule sealant further comprises a fluorine compound (E) and it is preferable that the dentinal tubule sealant further comprises silica particles (F).

It is also preferable that a dentin penetration inhibition ratio achieved when one side of a 700 $\mu$m thick bovine tooth disc is treated with the dentinal tubule sealant satisfies the following formula (1):

$$[1 - (\text{penetrated amount of a dentinal tubule-sealed bovine tooth disc})/(\text{penetrated amount of a dentinal tubule-unsealed bovine tooth disc})] \times 100 \geq 70 \ ... \ (I).$$

A dentinal tubule sealant that is used for sealing dentinal tubules by rubbing it into a dentin surface is a preferred embodiment of the present invention. A dentifrice comprising the dentinal tubule sealant is another preferred embodiment of the present invention. A tooth surface-treating material comprising the dentinal tubule sealant is another preferred embodiment of the present invention. A dentinal hypersensitivity inhibitor comprising the dentinal tubule sealant is another preferred embodiment of the present invention.

The above-described problems can be solved by providing a method for producing a dentinal tubule sealant, the method comprising mixing poorly-soluble calcium phosphate particles (A), a phosphorus-free calcium compound (B), and a liquid or aqueous paste comprising water (C) as a main component, wherein the poorly-soluble calcium phosphate particles (A) are at least one member selected from the group consisting of dicalcium phosphate anhydrous [$CaHPO_4$] particles, $\alpha$-tricalcium phosphate [$\alpha$-$Ca_3(PO_4)_2$] particles, $\beta$-tricalcium phosphate [$\beta$-$Ca_3(PO_4)_2$] particles, amorphous calcium phosphate [$Ca_3(PO_4)_2 \cdot nH_2O$] particles, calcium pyrophosphate [$Ca_2P_2O_7$] particles, calcium pyrophosphate dihydrate [$Ca_2P_2O_7 \cdot 2H_2O$] particles, octacalcium phosphate pentahydrate [$Ca_8H_2(PO_4)_6 \cdot 5H_2O$] particles, and dicalcium phosphate dihydrate [$CaHPO_4 \cdot 2H_2O$] particles, and the method comprises blending 30 to 76% by weight of the poorly-soluble calcium phosphate particles (A), 0.01 to 2% by weight of the phosphorus-free calcium compound (B), and 23 to 69% by weight of the liquid or aqueous paste comprising water (C) as a main component.

In this embodiment, it is preferable to add a liquid or aqueous paste comprising water (C) as a main component to a powder or nonaqueous paste comprising the poorly-soluble calcium phosphate particles (A) and the phosphorus-free calcium compound (B), and then mix them. It is preferable to add a liquid or aqueous paste comprising water (C) as a main component and also comprising the phosphorus-free calcium compound (B) to a powder or nonaqueous paste comprising the poorly-soluble calcium phosphate particles (A), and then mix them.

Moreover, in that embodiment, it is preferable to add a liquid or aqueous paste comprising water (C) as a main component and also comprising the poorly-soluble calcium phosphate particles (A) to a powder or nonaqueous paste comprising the phosphorus-free calcium compound (B), and then mix them. It is preferable to add a liquid or aqueous paste comprising water (C) as a main component and also comprising the phosphorus-free calcium compound (B) to a liquid or aqueous paste comprising water (C) as a main component and also comprising the poorly-soluble calcium phosphate particles (A), and then mix them. In these embodiments, it is also preferred that a mixing ratio (P/L) is from 0.5 to 3.

The above-described problems can be solved by providing a dentinal tubule sealant kit comprising a powder or nonaqueous paste comprising poorly-soluble calcium phosphate particles (A) and a phosphorus-free calcium compound (B) and a liquid or aqueous paste comprising water (C) as a main component.

The above-described problems can be solved by providing a dentinal tubule sealant kit comprising a powder or nonaqueous paste comprising poorly-soluble calcium phosphate particles (A), a powder or nonaqueous paste comprising a phosphorus-free calcium compound (B), and a liquid or aqueous paste comprising water (C) as a main component.

In these embodiments, it is preferred that the poorly-soluble calcium phosphate particles (A) have an average particle diameter of 0.8 to 7. 5 $\mu$m and the phosphorus-free calcium compound (B) has an average particle diameter of 0.3 to 12 $\mu$m.

The above-described problems can be solved by providing a dentinal tubule sealant kit comprising a powder or nonaqueous paste comprising poorly-soluble calcium phosphate particles (A), a phosphorus-free calcium compound (B) and an alkali metal salt of phosphoric acid (D), and a liquid or aqueous paste comprising water (C) as a main component.

The above-described problems can be solved by providing a dentinal tubule sealant kit comprising a powder or nonaqueous paste comprising poorly-soluble calcium phosphate particles (A) and an alkali metal salt of phosphoric acid (D), a powder or nonaqueous paste comprising a phosphorus-free calcium compound (B), and a liquid or aqueous paste comprising water (C) as a main component.

The above-described problems can be solved by providing a dentinal tubule sealant kit comprising a powder or nonaqueous paste comprising poorly-soluble calcium phosphate particles (A), a powder or nonaqueous paste comprising a phosphorus-free calcium compound (B) and an alkali metal salt of phosphoric acid (D), and a liquid or aqueous paste comprising water (C) as a main component.

In these embodiments, it is preferred that the poorly-soluble calcium phosphate particles (A) have an average particle diameter of 0.8 to 7.5 $\mu$m, the phosphorus-free calcium compound (B) has an average particle diameter of 0.3 to 12 $\mu$m, and the alkali metal salt of phosphoric acid (D) has an average particle diameter of 1 to 15 $\mu$m.

The above-described problems can be solved by providing a dentinal tubule sealant kit comprising a powder or nonaqueous paste comprising poorly-soluble calcium phosphate particles (A) and a liquid or aqueous paste comprising water (C) as a main component and also comprising a phosphorus-free calcium compound (B) .

In this embodiment, it is preferred that the poorly-soluble calcium phosphate particles (A) have an average particle diameter of 0.8 to 7.5 $\mu$m.

The above-described problems can be solved by providing a dentinal tubule sealant kit comprising a powder or nonaqueous paste comprising poorly-soluble calcium phosphate particles (A) and an alkali metal salt of phosphoric acid (D) and a liquid or aqueous paste comprising water (C) as a main component and also comprising a phosphorus-free calcium compound (B).

In this embodiment, it is preferred that the poorly-soluble calcium phosphate particles (A) have an average particle diameter of 0.8 to 7.5 $\mu$m and the alkali metal salt of phosphoric acid (D) has an average particle diameter of 1 to 15 $\mu$m.

The above-described problems can be solved by providing a dentinal tubule sealant kit comprising a liquid or aqueous paste comprising water (C) as a main component and also comprising poorly-soluble calcium phosphate particles (A) and a liquid or aqueous paste comprising water (C) as a main component and also comprising a phosphorus-free calcium compound (B).

The above-described problems can be solved by providing a dentinal tubule sealant kit comprising a liquid or aqueous paste comprising water (C) as a main component and also comprising poorly-soluble calcium phosphate particles (A), a liquid or aqueous paste comprising water (C) as a main component and a phosphorus-free calcium compound (B), and a powder or nonaqueous paste comprising an alkali metal salt of phosphoric acid (D).

In this embodiment, it is preferred that the average particle diameter of the alkali metal salt of phosphoric acid (D) is 1 to 15 $\mu$m.

The above-described problems can be solved by providing a dentinal tubule sealant kit comprising a powder or nonaqueous paste comprising a phosphorus-free calcium compound (B) and a liquid or aqueous paste comprising water (C) as a main component and also comprising poorly-soluble calcium phosphate particles (A) .

In this embodiment, it is preferred that the phosphorus-free calcium compound (B) has an average particle diameter of 0.3 to 12 $\mu$m.

The above-described problems can be solved by providing a dentinal tubule sealant kit comprising a powder or nonaqueous paste comprising a phosphorus-free calcium compound (B) and an alkali metal salt of phosphoric acid (D) and a liquid or aqueous paste comprising water (C) as a main component and also comprising poorly-soluble calcium phosphate particles (A).

In this embodiment, it is preferred that the phosphorus-free calcium compound (B) has an average particle diameter of 0.3 to 12 $\mu$m and the alkali metal salt of phosphoric acid (D) has an average particle diameter of 1 to 15 $\mu$m.

## EFFECT OF THE INVENTION

By the present invention, there is provided a dentinal tubule sealant capable of sealing dentinal tubules of an exposed dentin and also remineralizing the surrounding dentin after the sealing. This makes it possible to perform the therapy of hyperesthesia caused by opening of dentinal tubules, and also can impart caries resistance because the composition having sealed dentinal tubules will strengthen the tooth substance of the surrounding dentin.

## BRIEF DESCRIPTION OF THE DRAWINGS

[Fig. 1] A SEM photograph of a cross section of a bovine dentin in which dentinal tubules were sealed with a dentinal tubule sealant of the present invention in Example 8.

[Fig. 2] A SEM photograph (a) of a bovine dentin surface in which dentinal tubules were exposed and a SEM photograph (b) of a bovine dentin surface in which dentinal tubules were sealed with a dentinal tubule sealant of the present invention in Example 8.

## DETAILED DESCRIPTION OF THE INVENTION

The dentinal tubule sealant of the present invention contains 30 to 76% by weight of poorly-soluble calcium phosphate particles (A) that is at least one member selected from the group consisting of dicalcium phosphate anhydrous [$CaHPO_4$] particles (this may hereinafter be abbreviated to "DCPA"), $\alpha$-tricalcium phosphate [$\alpha$-$Ca_3(PO_4)_2$] particles, $\beta$-tricalcium phosphate [$\beta$-$Ca_3(PO_4)_2$] particles (this may hereinafter be abbreviated to $\beta$-TCP), amorphous calcium phosphate [$Ca_3(PO_4)_2 \cdot nH_2O$] particles, calcium pyrophosphate [$Ca_2P_2O_7$] particles, calcium pyrophosphate dihydrate [$Ca_2P_2O_7 \cdot 2H_2O$] particles, octacalcium phosphate pentahydrate [$Ca_8H_2(PO_4)_6 \cdot 5H_2O$] particles (this may hereinafter be abbreviated to OCP), and dicalcium phosphate dihydrate [$CaHPO_4 \cdot 2H_2O$] (this may hereinafter be abbreviated to DCPD), 0.001 to 4% by weight of a phosphorus-free calcium compound (B), and 23 to·69% by weight of water (C). This makes it possible to seal dentinal tubules of an exposed dentin and also to remineralize the surrounding dentin after the sealing. Although the action mechanism of this is not necessarily clear, the following mechanism is presumed.

The poorly-soluble calcium phosphate particles (A) to be used in the present invention have been selected from calcium phosphates having a solubility at around pH 7.0 being equal to or lower than that of DCPD and the solubility thereof in water is low. Accordingly, the poorly-soluble calcium phosphate particles (A) in the dentinal tubule sealant of the present invention hardly dissolve, and they exist in the form of particles. The phosphorus-free calcium compound (B) dissolves in water (C) to release calcium ion, thereby dissolving slightly the surface of the poorly-soluble calcium phosphate particles (A) to make it release calcium ion and phosphate ion, thereby forming HAp. If the dentinal tubule sealant of the present invention is applied to an exposed dentin surface, the poorly-soluble calcium phosphate particles (A) can enter directly into the dentinal tubule. At this time, it seems that calcium ion supplied by the phosphorus-free calcium compound (B), calcium ion having dissolved slightly from the surface of the poorly-soluble calcium phosphate particles (A), and phosphate ion react together to precipitate as HAp, which seems to combine the poorly-soluble calcium phosphate particles (A) together. It seems that the poorly-soluble calcium phosphate particles (A) having entered into the dentinal tubule are bound also to the dentinal tubule surface via the precipitated HAp, so that a strong lumpy sealant will be formed. This reaction is completed in a time as short as several minutes from the application of the dentinal tubule sealant of the present invention to the dentin.

Moreover, the dentinal tubule sealant of the present invention changes into HAp to the inside of the dentinal tubule sealant over several weeks by the action of a calcium ion and a phosphate ion in an oral cavity and thereby can densify and strengthen a sealing material. Furthermore, the dentinal tubule sealant serves as a release source of a calcium ion and a phosphate ion to strengthen the tooth substance of dentin surrounding a dentinal tubule, so that it can improve caries resistance as well.

The dentinal tubule sealant of the present invention needs to contain 30 to 76% by weight of the poorly-soluble calcium phosphate particles (A). When the content of the poorly-soluble calcium phosphate particles (A) is less than 30% by weight, excessively little insoluble components are present in the composition, so that it may become impossible to seal dentinal tubules sufficiently. The content of the poorly-soluble calcium phosphate particles (A) is preferably 40% by weight or more, more preferably 50% by weight or more. On the other hand, when the content of the poorly-soluble calcium phosphate particles (A) is more than 76% by weight, it becomes impossible to paste the composition to a sufficient degree because of a low content of a liquid agent component, and accordingly, operativity will deteriorate, so that the composition may become incapable of entering into dentinal tubules well. Moreover, the deposit of HAp in a short time is inhibited, so that a massive sealing material cannot be obtained and therefore it may become impossible to seal dentinal tubules. The content of the poorly-soluble calcium phosphate particles (A) is preferably 70% by weight or less, more preferably 63% by weight or less.

It is preferred that the average particle diameter of the poorly-soluble calcium phosphate particles (A) to be used in the present invention is 0.8 to 7.5 $\mu$m. When the average particle diameter is less than 0.8 $\mu$m, the particles dissolve in a liquid agent and hardly stay in dentinal tubules, so that sealability may deteriorate. Moreover, there is a possibility that the viscosity of a paste obtained by mixing with a liquid agent may become excessively high and there is a possibility that spreadability may deteriorate and, as a result, dentinal tubule sealability may deteriorate. More preferably, the average particle diameter of the poorly-soluble calcium phosphate particles (A) is 1 $\mu$m or more. On the other hand, when the average particle diameter is more than 7. 5 $\mu$m, the poorly-soluble calcium phosphate particles (A) cannot enter into dentinal tubules, so that sealability may deteriorate. Preferably, poorly-soluble calcium phosphate has an average particle diameter that is slightly larger than the diameter of dentinal tubules because it is poorly but slightly soluble in water. The average particle diameter of the poorly-soluble calcium phosphate particles (A) is more preferably 7 $\mu$m or less, even more preferably 6 $\mu$m or less, and particularly preferably 4 $\mu$m or less. The average particle diameter of the poorly-soluble calcium phosphate particles (A) is a value measured and calculated by using a laser diffraction type particle size distribution analyzer.

A method for producing the poorly-soluble calcium phosphate particles (A) having such an average particle diameter is not particularly restricted. While commercial products may be used if available, it is often preferable to further grind a commercially available product. In such a case, a grinding machine, such as a ball mill, a pestle and mortar machine and a jet mill, can be used. The poorly-soluble calcium phosphate particles (A) can also be obtained by grinding a raw material powder of poorly-soluble calcium phosphate together with such a liquid medium as alcohol by the use of a pestle and mortar machine, a ball mill, or the like to prepare a slurry, and drying the obtained slurry. As the grinding machine in this process, a ball mill is preferably used. As the material of its pot and balls, alumina or zirconia is preferably used. Moreover, poorly-soluble calcium phosphate particles (A) having a particle diameter on the nano scale can be obtained by spray-drying a dilute solution of poorly-soluble calcium phosphate.

The phosphorus-free calcium compound (B) to be used in the present invention is not particularly restricted, and examples thereof include calcium hydroxide [$Ca(OH)_2$], calcium oxide [$CaO$], calcium chloride [$CaCl_2$], calcium nitrate [$Ca(NO_3)_2 \cdot nH_2O$], calcium acetate [$Ca(CH_3CO_2)_2 \cdot nH_2O$] , calcium lactate [$C_6H_{10}CaO_6$], calcium citrate [$Ca_3(C_6H_5O_7)_2 \cdot nH_2O$], calcium metasilicate [$CaSiO_3$], dicalcium silicate [$Ca_2SiO_4$], tricalcium silicate [$Ca_3SiO_5$], and calcium carbonate [$CaCO_3$]; one member or two or more members out of such compounds are to be used. Especially, in view of HAp depositability, calcium hydroxide, calcium oxide, calcium metasilicate, dicalcium silicate, and tricalcium silicate are preferred, and calcium hydroxide is more preferred.

The dentinal tubule sealant of the present invention needs to contain the phosphorus-free calcium compound (B) in a content of 0.01 to 2% by weight. When the content of the phosphorus-free calcium compound (B) is less than 0.001% by weight, deposition of HAp hardly occurs on poorly-soluble calcium phosphate particles (A) and therefore poorly-soluble calcium phosphate particles (A) having entered into a dentinal tubule cannot be combined together, so that it is impossible to form a massive sealing material in dentinal tubules. Therefore, the poorly-soluble calcium phosphate particles (A) having entered into dentinal tubules dissolve slowly and flow away, so that the dentinal tubules open again. When the content of the phosphorus-free calcium compound (B) is 0.01% by weight or more, it can dissolve the surface of the poorly-soluble calcium phosphate particle (A) and deposit HAp. The content of the phosphorus-free calcium compound (B) is 0.01% by weight or more. When the content of the phosphorus-free calcium compound (B) is more than 4.0% by weight, the amount of a soluble component in the composition becomes excessively large and therefore a void is formed within a sealing material formed, so that it may become impossible to seal dentinal tubules well. The content of the phosphorus-free calcium compound (B) is 2% by weight or less.

Moreover, the phosphorus-free calcium compound (B) to be used in the present invention may be blended either in the form of a powder or in the form of a liquid agent; it has a dentinal tubule sealing effect in either case. It is noted that to add and incorporate the phosphorus-free calcium compound (B) intactly in the form of a powder is preferred because this allows the phosphorus-free calcium compound (B) to dissolve after entering into a dentinal tubule and thus the calcium ion concentration around poorly-soluble calcium phosphate particles (A) becomes high, and the surface of the poorly-soluble calcium phosphate particles (A) is dissolved to deposit HAp, so that it becomes easier to form a massive sealing material.

Preferably, the Ca/P ratio of the poorly-soluble calcium phosphate particle (A) and the phosphorus-free calcium compound (B) to be used in the present invention in total is 0.9 to 1.25. Usually, the Ca/P ratio is known to be desirable to be adjusted to 1.67, which is equal to the Ca/P ratio in HAp. However, in the dentinal tubule sealant of the present invention, only the surface of the poorly-soluble calcium phosphate particles (A) dissolves, so that phosphate ions and calcium ions are emitted. Subsequently, the calcium ions and the phosphate ions react together with calcium ions derived from the phosphorus-free calcium compound (B) to deposit HAp and form a massive sealing material. When calcium ion increases too much, the dissolved amount of poorly-soluble calcium phosphate particles (A) becomes large and thus voids are formed in a massive sealing material, so that it may become impossible to seal dentinal tubules well. More preferably, the Ca/P ratio is 1.2 or less. On the other hand, in order to obtain a denser massive sealing material, the Ca/P ratio is more preferably 1 or more.

It is preferred that the average particle diameter of the phosphorus-free calcium compound (B) is 0.3 to 12 $\mu$m. When the average particle diameter is less than 0.3 $\mu$m, the calcium ion concentration in the composition becomes high before entry into a dentinal tubule because of excessively fast dissolution in a liquid agent and change in the nature into hydroxyapatite on a surface of poorly-soluble calcium phosphate begins before entry into a dentinal tubule, so that a massive sealing material becomes difficult to form. The average particle diameter is more preferably 0.7 $\mu$m or more. It is even more preferably 2 $\mu$m or more. On the other hand, in the case that the average particle diameter of the phosphorus-free calcium compound (B) is more than 12 $\mu$m, the phosphorus-free calcium compound (B) is difficult to dissolve in a liquid agent. Moreover, the phosphorus-free calcium compound (B) cannot enter into dentinal tubules, so that sealability may deteriorate. The average particle diameter of the phosphorus-free calcium compound (B) is more preferably 9.0 $\mu$m or less. The average particle diameter of the phosphorus-free calcium compound (B) is a value measured and calculated by using a laser diffraction type particle size distribution analyzer.

The method for producing the phosphorus-free calcium compound (B) having such an average particle diameter can be performed in a similar manner to the poorly-soluble calcium phosphate particle (A) described above.

The dentinal tubule sealant of the present invention needs to further include 23 to 69% by weight of water (C) in addition to the poorly-soluble calcium phosphate particles (A) and the phosphorus-free calcium compound (B). When the content of the water (C) is less than 23% by weight, it becomes impossible to paste the composition to a sufficient degree because of a low content of a liquid agent component, and accordingly, operativity will deteriorate, so that the composition may become incapable of entering into dentinal tubules well. Moreover, the deposit of HAp in a short time is inhibited, so that a massive sealing material cannot be obtained and therefore it may become impossible to seal dentinal tubules. The content of the water (C) is preferably 25% by weight or more, more preferably 28% by weight or more. On the other hand, in the event that the content of the water (C) is more than 69% by weight, it is impossible to seal dentinal tubules because the content of insoluble components is too little. The content of the water (C) is preferably 60% by weight or less, more preferably 50% by weight or less.

Preferably, the dentinal tubule sealant of the present invention further includes an alkali metal salt of phosphoric acid (D). The alkali metal salt of phosphoric acid (D) gives a phosphate ion when HAp deposits, thereby increasing the deposition rate of HAp. If there is a phosphoric ion released from an alkali metal salt of phosphoric acid (D) when poorly-soluble calcium phosphate particles (A) and a phosphorus-free calcium compound (B) enter into a dentinal tubule and form a massive sealing material, the deposition rate of HAp increases and thus the massive sealing material is formed more densely, so that the dentinal tubule sealing ratio can be increased. The alkali metal salt of phosphoric acid (D) to be used in the present invention is not particularly restricted, and examples thereof include disodium hydrogen phosphate, dipotassium hydrogen phosphate, lithium dihydrogen phosphate, sodium dihydrogen phosphate, potassium dihydrogen phosphate, trisodium phosphate, tripotassium phosphate, and hydrates thereof, among which one salt or two or more salts are used. Particularly, from the viewpoint of safety or easiness of obtaining a raw material with high purity, it is preferred that the alkali metal salt of phosphoric acid (D) is disodium hydrogen phosphate and/or sodium dihydrogen phosphate.

Preferably, the content of the alkali metal salt of phosphoric acid (D) is 0.1 to 25% by weight. When the loading of the alkali metal salt of phosphoric acid (D) is less than 0.1% by weight, the deposition rate of HAp becomes slow, so that the dentinal tubule sealing ratio may decrease a little though a massive sealing material is formed. The content of the alkali metal salt of phosphoric acid (D) is more preferably 0.3% by weight or more, even more preferably 1% by weight or more. On the other hand, when the content of the alkali metal salt of phosphoric acid (D) is more than 25% by weight, the amount of a soluble component in the composition becomes excessively large and therefore a void is formed within a dentinal tubule sealing material formed, so that it may become impossible to seal dentinal tubules well. The content of the alkali metal salt of phosphoric acid (D) is more preferably 20% by weight or less, even more preferably 15% by weight or less, and particularly preferably 8% by weight or less.

Moreover, the alkali metal salt of phosphoric acid (D) to be used in the present invention may be blended either in the form of a powder or in the form of a liquid agent; it has a dentinal tubule sealing effect in either case. However, rather by being added and incorporated in a powder form, the alkali metal salt of phosphoric acid (D) can enter into dentinal tubules and then dissolve, so that the phosphate ion concentration around poorly-soluble calcium phosphate particles (A) becomes higher. This is preferable because as a result of this, a HAp deposition rate increases, so that a massive sealing material becomes denser and thus a dentin penetration inhibition ratio increases.

Preferably, the average particle diameter of the alkali metal salt of phosphoric acid (D) is 1 to 15 $\mu$m. When the average particle diameter is less than 1 $\mu$m, the phosphate ion concentration in the composition becomes high before entry into a dentinal tubule because of excessively fast dissolution in a liquid agent and change in the nature of hydroxyapatite on a surface of poorly-soluble calcium phosphate begins before entry into a dentinal tubule, so that a massive sealing material becomes difficult to form. The secondary aggregation of particles of the alkali metal salt of phosphoric acid occurs, so that the dispersibility of the particles with other particles to be mixed simultaneously will deteriorate. More preferably, the average particle diameter is 2 m or more. On the other hand, when the average particle diameter is more than 15 $\mu$m, the alkali metal salt of phosphoric acid (D) becomes less soluble in a liquid agent and cannot enter into dentinal tubules, so that sealability will deteriorate. Since an alkali metal salt of phosphoric acid can dissolve in water, it is preferable for the alkali metal salt of phosphoric acid to have an average particle diameter larger than the diameter of a dentinal tubule. More preferably, the average particle diameter of the alkali metal salt of phosphoric acid (D) is 8 $\mu$m or less. The average particle diameter of the alkali metal salt of phosphoric acid (D) is a value measured and calculated by using a laser diffraction type particle size distribution analyzer.

The method for producing the alkali metal salt of phosphoric acid (D) having such an average particle diameter can be performed in a similar manner to the poorly-soluble calcium phosphate particle (A) described above.

Preferably, the dentinal tubule sealant of the present invention further includes a fluorine compound (E) . This enables it to impart acid resistance to a sealing material formed and also promote remineralization of the surrounding dentin. The fluorine compound (E) to be used in the present invention, is not particularly restricted, and examples thereof include sodium fluoride, potassium fluoride, ammonium fluoride, lithium fluoride, cesium fluoride, magnesium fluoride, calcium fluoride, strontium fluoride, barium fluoride, copper fluoride, zirconium fluoride, aluminum fluoride, stannous fluoride, sodium monofluorophosphate, potassium monofluorophosphorate, hydrofluoric acid, titanium sodium fluoride, titanium potassium fluoride, hexylamine hydrofluoride, laurylamine hydrofluoride, glycine hydrofluoride, alanine hydrofluoride, fluorosilanes, and diamine silver fluoride. Among these, sodium fluoride, sodium monofluorophosphate, and stannous fluoride are suitably used from the viewpoint that an acid-resistant sealant forming effect is high.

The used amount of the fluorine compound (E) to be used in the present invention is not particularly limited and it is preferred that 0.01 to 3% by weight of the fluorine compound (E) in terms of fluorine ion is contained. When the used amount of the fluorine compound (E) in terms of fluoride ion is less than 0.01% by weight, there is a possibility that the acid-resistant sealant forming effect and the effect of promoting remineralization may deteriorate, and it is more preferred that the used amount is 0.05% by weight or more. On the other hand, when the used amount of the converted fluoride ions of the fluorine compound (E) exceeds 3% by weight, there is a possibility that safety may be impaired, and it is more preferred that the used amount is 1% by weight or less.

Preferably, the dentinal tubule sealant of the present invention further includes silica particles (F). The operativity of the dentinal tubule sealant of the present invention obtained can thereby be improved. From the viewpoint of such improvement in operativity, silica particles (F) having a primary particle diameter of 0.001 to 0.1 $\mu$m is preferably used. Examples of commercially available products include "Aerosil OX50", "Aerosil 50", "Aerosil 200", "Aerosil 380", "Aerosil R972", and "Aerosil 130" (all are commercial names and produced by Nippon Aerosil Co., Ltd.).

The used amount of the silica particles (F) to be used in the present invention is not particularly limited, and it is preferred that the silica particles (F) are contained in an amount of 0.1 to 10% by weight. When the content of the silica particles (F) is less than 0.1% by weight, operativity may deteriorate, and the content is more preferably 0.3% by weight or more. On the other hand, when the content of the silica particles (F) is more than 10% by weight, the bulk density of a powder decreases excessively, so that operativity will deteriorate and also the viscosity of a paste prepared may increase; the content is more preferably 5% by weight or less.

The dentinal tubule sealant of the present invention may further include filler other than the silica particles (F). Regarding the filler, a single filler may be incorporated or alternatively two or more fillers may be incorporated in combination. Examples of the filler include minerals containing silica as a base, such as kaolin, clay, mica, and mica; and ceramics and glass containing silica as a base and also containing $Al_2O_3$, $B_2O_3$, $TiO_2$, $ZrO_2$, $BaO$, $La_2O_3$, $SrO$, $ZnO$, $CaO$, $P_2O_5$, $Li_2O$, $Na_2O$, etc. As the glass, lanthanum glass, barium glass, strontium glass, soda glass, lithium borosilicate glass, zinc glass, fluoroaluminosilicate glass, borosilicated glass, and bioglass are suitably used. Crystal quartz, alumina, titanium oxide, yttrium oxide, zirconia, barium sulfate, aluminum hydroxide, and ytterbium fluoride are also suitably used.

The dentinal tubule sealant of the present invention may include components other than the poorly-soluble calcium phosphate particles (A), the phosphorus-free calcium compound (B), the water (C), the alkali metal salt of phosphoric acid (D), the fluorine compound (E), and the silica particles (F) as far as the effect of the present invention is not damaged. For example, soluble calcium phosphate can also be incorporated according to need. Specific examples of the soluble calcium phosphate include tetracalcium phosphate, monocalcium phosphate anhydrous, and calcium dihydrogen pyrophosphate. Besides, a thickener may also be incorporated. Specific examples of the thickener may be one or two or more species selected from among carboxymethylcellulose, sodium carboxymethylcellulose, hydroxypropylcellulose, hydroxypropylmethylcellulose, polyvinyl alcohol, polyethylene glycol, polyacrylic acid, polystyrene sulfonic acid, polystyrene sulfonic acid salts, polyglutamic acid, polyglutamic acid salts, polyaspartic acid, polyaspartic acid salts, polyL-lysin, polyL-lysin salts, starch other than cellulose, alginic acid, alginic acid salts, carrageenan, guar gum, xanthan gum, cellulose gum, hyaluronic acid, hyaluronic acid salts, pectin, pectin salts, polysaccharides such as chitin and chitosan, acidic polysaccharide esters such as propylene glycol alginate, and polymers such as proteins, e.g. collagen, gelatin and their derivatives. From aspects of solubility in water and viscosity, at least one species selected from sodium carboxymethylcellulose, hydroxypropylcellulose, hydroxypropylmethylcellulose, alginic acid, alginic acid salts, chitosan, polyglutamic acid and polyglutamic acid salts is preferred. The thickener may be blended with a powder, and may be blended with a liquid agent, and also may be blended with a paste under mixing.

According to need, polyhydric alcohols, such as glycerol, ethylene glycol, propylene glycol, and diglycerol, sugar alcohols, such as xylitol, sorbitol, and erythritol, polyethers, such as polyethylene glycol and polypropylene glycol, artificial sweeteners, such as aspartame, acesulfame potassium, liquorice extract, saccharin, and saccharin sodium, and so on may also be added. Moreover, all pharmacologically acceptable drugs can be blended. For example, antibacterial agents typified by cetyl pyridinium chloride etc., disinfectants, anticancer drugs, antibiotics, blood circulation improvers, such as Actosin and PEG1, growth factors, such as bFGF, PDGF, and BMP, cells which promote hard tissue formation, such as osteoblasts, odontoblasts, and anaplastic bone marrow derived stem cells, embryonic stem (ES) cells, induced pluripotent stem (iPS) cells produced by dedifferentiating differentiated cells such as fibroblasts by gene introduction and cells produced by differentiating the foregoing can be blended.

In the present invention, a dentinal tubule sealant in paste form can be obtained by mixing poorly-soluble calcium phosphate particles (A), a phosphorus-free calcium compound (B), and a liquid or aqueous paste comprising water (C) as a main component. Since this dentinal tubule sealant in paste form containing the water (C) starts to develop a reaction of immediate conversion into HAp, it is preferred to be prepared just prior to use at a medical site. A mixing operation is not particularly restricted, and manual mixing and mixing with a static mixer are preferably adopted.

In the present invention, the method for obtaining a dentinal tubule sealant is not particularly restricted. The dentinal tubule sealant in paste form can be obtained by adding a liquid or aqueous paste comprising water (C) as a main component to a powder or nonaqueous paste comprising poorly-soluble calcium phosphate particles (A) and a phosphorus-free calcium compound (B), and then mixing them. The dentinal tubule sealant in paste form can be obtained also by mixing a powder or nonaqueous paste comprising poorly-soluble calcium phosphate particles (A), a powder or nonaqueous paste comprising a phosphorus-free calcium compound (B), and a liquid or aqueous paste comprising water (C) as a main component. The dentinal tubule sealant in paste form can be obtained also by mixing a liquid or aqueous paste comprising water (C) as a main component and also comprising poorly-soluble calcium phosphate particles (A) with a powder or nonaqueous paste comprising a phosphorus-free calcium compound (B) . The dentinal tubule sealant in paste form can be obtained also by mixing a liquid or aqueous paste comprising water (C) as a main component and also comprising a phosphorus-free calcium compound (B) to a powder or nonaqueous paste comprising poorly-soluble calcium phosphate particles (A). The dentinal tubule sealant in paste form can be obtained also by mixing a liquid or aqueous paste comprising water (C) as a main component and also comprising poorly-soluble calcium phosphate particles (A) with a liquid or aqueous paste comprising water (C) as a main component and also comprising a phosphorus-free calcium compound (B).

The method of mixing an alkali metal salt of phosphoric acid (D) is not particularly restricted. The dentinal tubule sealant in paste form can be obtained by adding a liquid or aqueous paste comprising water (C) as a main component to a powder or nonaqueous paste comprising poorly-soluble calcium phosphate particles (A), a phosphorus-free calcium compound (B), and an alkali metal salt of phosphoric acid (D), and then mixing them. The dentinal tubule sealant in paste form can be obtained by adding a liquid or aqueous paste comprising water (C) as a main component and also comprising an alkali metal salt of phosphoric acid (D) to a powder or nonaqueous paste comprising poorly-soluble calcium phosphate particles (A) and a phosphorus-free calcium compound (B), and then mixing them. The dentinal tubule sealant in paste form can be obtained also by mixing a powder or nonaqueous paste comprising poorly-soluble calcium phosphate particles (A) and an alkali metal salt of phosphoric acid (D), a powder or nonaqueous paste comprising a phosphorus-free calcium compound (B), and a liquid or aqueous paste comprising water (C) as a main component. The dentinal tubule sealant in paste form can be obtained also by mixing a powder or nonaqueous paste comprising poorly-soluble calcium phosphate particles (A), a powder or nonaqueous paste comprising a phosphorus-free calcium compound (B) and an alkali metal salt of phosphoric acid (D), and a liquid or aqueous paste comprising water (C) as a main component. The dentinal tubule sealant in paste form can be obtained also by mixing a powder or nonaqueous paste comprising poorly-soluble calcium phosphate particles (A), a powder or nonaqueous paste comprising a phosphorus-free calcium compound (B), and a liquid or aqueous paste comprising water (C) as a main component and also comprising an alkali metal salt of phosphoric acid (D). The dentinal tubule sealant in paste form can be obtained also by mixing a powder or nonaqueous paste comprising a phosphorus-free calcium compound (B) and an alkali metal salt of phosphoric acid (D) with a liquid or aqueous paste comprising water (C) as a main component and also comprising poorly-soluble calcium phosphate particles (A). The dentinal tubule sealant in paste form can be obtained also by mixing a powder or nonaqueous paste comprising a phosphorus-free calcium compound (B) with a liquid or aqueous paste comprising water (C) as a main component and also comprising poorly-soluble calcium phosphate particles (A) and an alkali metal salt of phosphoric acid (D). The dentinal tubule sealant in paste form can be obtained also by mixing a powder or nonaqueous paste comprising poorly-soluble calcium phosphate particles (A) and an alkali metal salt of phosphoric acid (D) with a liquid or aqueous paste comprising water (C) as a main component and also comprising a phosphorus-free calcium compound (B). The dentinal tubule sealant in paste form can be obtained also by mixing a powder or nonaqueous paste comprising poorly-soluble calcium phosphate particles (A) with a liquid or aqueous paste comprising water (C) as a main component and also comprising a phosphorus-free calcium compound (B) and an alkali metal salt of phosphoric acid (D). The dentinal tubule sealant in paste form can be obtained also by mixing a liquid or aqueous paste comprising water (C) as a main component and also comprising poorly-soluble calcium phosphate particles (A), a liquid or aqueous paste comprising water (C) as a main component and also comprising a phosphorus-free calcium compound (B), and a powder or nonaqueous paste comprising an alkali metal salt of phosphoric acid (D). The dentinal tubule sealant in paste form can be obtained also by mixing a liquid or aqueous paste comprising water (C) as a main component and also comprising poorly-soluble calcium phosphate particles (A) and an alkali metal salt of phosphoric acid (D) with a liquid or aqueous paste comprising water (C) as a main component and also comprising a phosphorus-free calcium compound (B). The dentinal tubule sealant in paste form can be obtained also by mixing a liquid or aqueous paste comprising water (C) as a main component and also comprising poorly-soluble calcium phosphate particles (A) with a liquid or aqueous paste comprising water (C) as a main component and also comprising a phosphorus-free calcium compound (B) and an alkali metal salt of phosphoric acid (D).

From the viewpoint of obtaining good operativity and obtaining a denser massive sealing material, it is preferable to add a liquid or aqueous paste comprising water (C) as a main component to a powder or nonaqueous paste comprising poorly-soluble calcium phosphate particles (A) and a phosphorus-free calcium compound (B), and then mix them. It is preferable to add a liquid or aqueous paste comprising water (C) as a main component and also comprising a phosphorus-free calcium compound (B) to a powder or nonaqueous paste comprising poorly-soluble calcium phosphate particles (A), and then mix them. It is preferable to add a liquid or aqueous paste comprising water (C) as a main component and also comprising poorly-soluble calcium phosphate particles (A) to a powder or nonaqueous paste comprising a phosphorus-free calcium compound (B), and then mix them. It is preferable to add a liquid or aqueous paste comprising water (C) as a main component and also comprising a phosphorus-free calcium compound (B) to a liquid or aqueous paste comprising water (C) as a main component and also comprising poorly-soluble calcium phosphate particles (A), and then mix them.

In these methods, the operation in preparation by mixing just before use is simple and easy. The solvent other than water to be used for the nonaqueous paste is not particularly restricted, and examples thereof include polyhydric alcohols, such as glycerol, ethylene glycol, propylene glycol, and diglycerol, and polyethers, such as polyethylene glycol and polypropylene glycol. In the above-described methods for producing a dentinal tubule sealant, when a phosphorus-free calcium compound (B) is contained in a powder or nonaqueous paste, calcium hydroxide, calcium oxide, calcium metasilicate, dicalcium silicate, and tricalcium silicate are suitably used as the phosphorus-free calcium compound (B).

Since the presence of water (C) allows a reaction in which a dentinal tubule sealant comprising poorly-soluble calcium phosphate particles (A) and a phosphorus-free calcium compound (B) is converted into HAp promptly to occur, it is impossible to store poorly-soluble calcium phosphate particles (A), a phosphorus-free calcium compound (B), and a liquid or aqueous paste comprising water (C) as a main component after mixing them in advance. A dentinal tubule sealant kit comprising a powder or nonaqueous paste comprising poorly-soluble calcium phosphate particles (A) and a phosphorus-free calcium compound (B) and a liquid or aqueous paste comprising water (C) as a main component wherein the poorly-soluble calcium phosphate particles (A) are at least one member selected from the group consisting of dicalcium phosphate anhydrous [CaHPO$_4$] particles, α-tricalcium phosphate [α-Ca$_3$(PO$_4$)$_2$] particles, β-tricalcium phosphate [β-Ca$_3$(PO$_4$)$_2$] particles, amorphous calcium phosphate [Ca$_3$(PO$_4$)$_2$•nH$_2$O] particles, calcium pyrophosphate [Ca$_2$P$_2$O$_7$] particles, calcium pyrophosphate dihydrate [Ca$_2$P$_2$O$_7$•2H$_2$O] particles, octacalcium phosphate pentahydrate [Ca$_8$H$_2$(PO$_4$)$_6$•5H$_2$O] particles, and dicalcium phosphate dihydrate [CaHPO$_4$•2H$_2$O] particles, and the kit contains 30 to 76% by weight of the poorly-soluble calcium phosphate particles (A), 0.01 to 2% by weight of the phosphorus-free calcium compound (B), and 23 to 69% by weight of the water (C), is a further embodiment of the present invention. A dentinal tubule sealant kit comprising a powder or nonaqueous paste comprising poorly-soluble calcium phosphate particles (A), a powder or nonaqueous paste comprising a phosphorus-free calcium compound (B), and a liquid or aqueous paste comprising water (C) as a main component as specified above is a further embodiment of the present invention. A dentinal tubule sealant kit comprising a powder or nonaqueous paste comprising poorly-soluble calcium phosphate particles (A) and a liquid or aqueous paste comprising water (C) as a main component and also comprising a phosphorus-free calcium compound (B) specified above is a further embodiment of the present invention. A dentinal tubule sealant kit comprising a liquid or aqueous paste comprising water (C) as a main component and also comprising poorly-soluble calcium phosphate particles (A) and a liquid or aqueous paste comprising water (C) as a main component and also comprising a phosphorus-free calcium compound (B) as specified above is a further embodiment of the present invention. A dentinal tubule sealant kit comprising a powder or nonaqueous paste comprising a phosphorus-free calcium compound (B) and a liquid or aqueous paste comprising water (C) as a main component and also comprising poorly-soluble calcium phosphate particles (A) as specified above is a further embodiment of the present invention.

When the dentinal tubule sealant of the present invention includes an alkali metal salt of phosphoric acid (D), a dentinal tubule sealant kit comprising a powder or nonaqueous paste comprising poorly-soluble calcium phosphate particles (A), a phosphorus-free calcium compound (B), and an alkali metal salt of phosphoric acid (D), and a liquid or aqueous paste comprising water (C) as a main component is one preferred embodiment of the present invention. A dentinal tubule sealant kit comprising a powder or nonaqueous paste comprising poorly-soluble calcium phosphate particles (A) and an alkali metal salt of phosphoric acid (D), a powder or nonaqueous paste comprising a phosphorus-free calcium compound (B), and a liquid or aqueous paste comprising water (C) as a main component is one preferred embodiment of the present invention. A dentinal tubule sealant kit comprising a powder or nonaqueous paste comprising poorly-soluble calcium phosphate particles (A), a powder or nonaqueous paste comprising a phosphorus-free calcium compound (B) and an alkali metal salt of phosphoric acid (D), and a liquid or aqueous paste comprising water (C) as a main component is one preferred embodiment of the present invention. A dentinal tubule sealant kit comprising a powder or nonaqueous paste comprising poorly-soluble calcium phosphate particles (A) and an alkali metal salt of phosphoric acid (D) and a liquid or aqueous paste comprising water (C) as a main component and also comprising a phosphorus-free calcium compound (B) is one preferred embodiment of the present invention. A dentinal tubule sealant kit comprising a liquid or aqueous paste comprising water (C) as a main component and also comprising poorly-soluble calcium phosphate particles (A), a liquid or aqueous paste comprising water (C) as a main component and a phosphorus-free calcium compound (B), and a powder or nonaqueous paste comprising an alkali metal salt of phosphoric acid (D) is one preferred embodiment of the present invention. A dentinal tubule sealant kit comprising a powder or nonaqueous paste comprising a phosphorus-free calcium compound (B) and an alkali metal salt of phosphoric acid (D) and a liquid or aqueous paste comprising water (C) as a main component and also comprising poorly-soluble calcium phosphate particles (A) is one preferred embodiment of the present invention.

In these methods, the operation in preparation by mixing just before use is simple and easy. The solvent other than water (C) to be used for the nonaqueous paste is not particularly restricted, and examples thereof include polyhydric alcohols, such as glycerol, ethylene glycol, propylene glycol, and diglycerol, and polyethers, such as polyethylene glycol and polypropylene glycol. In the above-described dentinal tubule sealant kits, when a phosphorus-free calcium compound (B) is contained in a powder or nonaqueous paste, calcium hydroxide, calcium oxide, calcium metasilicate, dicalcium silicate, and tricalcium silicate are suitably used as the phosphorus-free calcium compound (B).

In the present invention, a dentinal tubule sealant can be obtained by mixing a powder and/or a nonaqueous paste with a liquid and/or an aqueous paste. Preferably, the mixing ratio (P/L) of the powder and/or the nonaqueous paste to the liquid and/or the aqueous paste is 0.5 to 3. When the P/L ratio is less than 0.5, the content of a powder component becomes low, so that it may become impossible to seal dentinal tubules. The P/L ratio is more preferably 0.6 or more, even more preferably 0.8 or more. When the P/L ratio is more than 3, it becomes impossible to paste the composition to a sufficient degree because of an excessively low content of a liquid component, and accordingly, operativity will deteriorate, so that the composition may become incapable of entering into dentinal tubules well. Moreover, the deposit of HAp in a short time is inhibited, so that a massive sealing material cannot be obtained and therefore it may become impossible to seal dentinal tubules. The P/L ratio is more preferably 2.2 or less, even more preferably 2 or less. Since poorly-soluble calcium phosphate particles (A) hardly dissolve and exist as a powder even if being incorporated into a liquid or an aqueous paste, the poorly-soluble calcium phosphate particles (A) are always calculated as a powder (P).

It is preferred for the dentinal tubule sealant of the present invention that a dentin penetration inhibition ratio achieved when one side of a 700 μm thick bovine tooth disc is treated with the dentinal tubule sealant satisfies the following formula (I). The dentinal tubule sealant of the present invention that satisfies the following formula (I) can seal dentinal tubules of an exposed dentin and also can remineralize the surrounding dentin. This makes it possible to perform the therapy of hyperesthesia caused by opening of dentinal tubules and, and also can impart caries resistance because the dentinal tubule sealant having sealed dentinal tubules will strengthen the tooth substance of the surrounding dentin.

$$[1 - (\text{penetrated amount of a dentinal tubule-sealed bovine tooth disc})/(\text{penetrated amount of a dentinal tubule-unsealed bovine tooth disc})] \times 100 \geq 70 \ \ldots \ (I).$$

The dentin penetration inhibition ratio is more preferably 75% or more, even more preferably 80% or more, particularly preferably 85% or more.

The dentinal tubule sealant of the present invention is suitably used for various applications such as a tooth surface-treating material and a dentifrice. That is, suitable embodiments of the present invention include a tooth surface-treating material comprising a dentinal tubule sealant and a dentifrice comprising a dentinal tubule sealant. Moreover, the hyperesthesia caused by opening of dentinal tubules can be treated by the use of the dentinal tubule sealant of the present invention, and from such a point of view, a dentinal hypersensitivity inhibitor comprising a dentinal tubule sealant is a preferred embodiment of the present invention. Since a reaction in which poorly-soluble calcium phosphate particles (A) and a phosphorus-free calcium compound (B) are promptly converted into HAp occurs particularly in the presence of water, an embodiment in which the agent is mixed appropriately with a liquid agent just prior to use, such as a tooth surface-treating material, is preferred. Therefore, the tooth surface-treating material comprising a dentinal tubule sealants is a more preferred embodiment of the present invention.

In order to make a composition reach into dentinal tubules, it is desirable for the dentinal tubule sealant of the present invention to apply a composition to a dentin surface and then perform an operation of rubbing the applied composition into dentinal tubules with a microbrush, a cotton swab, a rubber cup, or the like. The rubbing operation may be merely rubbing the dentin surface with a microbrush or the like for about 30 seconds, and a massive sealing material can thereby be formed to a depth of about 10 μm within dentinal tubules. The present inventors have confirmed that if a composition is applied to an exposed dentin and then is left at rest for several minutes, the composition never enters into a dentinal tubule having a diameter of about 2 μm and only a thin hydroxyapatite layer is formed on a dentin surface, and the formed hydroxyapatite layer will come off easily because it is bonded to a tooth substance weakly. Accordingly, a dentinal tubule sealant that is one to be used for sealing dentinal tubules by rubbing it into dentinal tubules is a preferred embodiment of the present invention. Moreover, a method for inhibiting dentinal hypersensitivity by rubbing such a dentinal tubule sealant into a dentin surface is also a preferred embodiment of the present invention.

In the present invention, since dentinal tubules will be sealed with a dentinal tubule sealant before applying a dental adhesive composition to a dentin surface, it becomes possible to suppress pains, hyperesthesia, and so on, and since solid components having adhered to a dentin surface can be removed by scrubbing using water, the adhesive property of a dental adhesive composition to a dentin surface becomes good. Accordingly, it is possible to provide a dental treatment method characterized in that the dentinal tubule sealant is rubbed into a dentin surface and then the dentin surface is scrubbed using water. Preferably, there can be provided a dental treatment method using a dentinal tubule sealant that is to be rubbed into a dentin surface to form a massive sealing material within dentinal tubules, wherein the method comprises rubbing the dentinal tubule sealant into a dentin surface to form a massive sealing material within dentinal tubules, and then removing a solid component adhering to the dentin surface by scrubbing using water. Moreover, the present invention can provide a dental treatment method that comprises rubbing a dentinal tubule sealant into a dentin surface, and then scrubbing the dentin surface by using water, and then applying and curing a dental adhesive composition. Preferably, there can be provided a dental treatment method using a dentinal tubule sealant that is to be applied to a dentin surface to form a massive sealing material within dentinal tubules, wherein the method comprises rubbing the dentinal tubule sealant into a dentin surface to form a massive sealing material within dentinal tubules, and then removing a solid component adhering to the dentin surface by scrubbing using water, and then applying and curing a dental adhesive composition on the dentin surface from which the solid component have been removed. The dentinal tubule sealant of the present invention is required that a solid component adhering to a dentin surface can be removed easily using water. If the fast curability of a dentinal tubule sealant becomes excessively high, this is undesirable because the dentinal tubule sealant is fixed firmly to a dentin surface and becomes difficult to be removed by scrubbing using water.

EXAMPLES

The present invention is explained below more concretely by way of Examples. In the Examples, regarding the average particle diameter of poorly-soluble calcium phosphate particles (A), particles of a phosphorus-free calcium compound (B), and particles of an alkali metal salt of phosphoric acid (D), measurement was conducted using a laser diffraction type particle size distribution analyzer ("SALD-2100" manufactured by Shimadzu Corporation), and a median diameter calculated from the result of the measurement was defined as the average particle diameter.

[Dentin penetration inhibition ratio evaluation]

(1) Production of bovine tooth for dentin penetration inhibition ratio evaluation

A cheek-side dentin of a healthy bovine incisor tooth was trimmed with #80, #1000 sand papers by using a rotary grinder, so that a dentin disc about 1.5 cm in diameter and 0.9 mm in thickness was produced. The surface of the bovine tooth disc was further polished with wrapping films (#1200, #3000, #8000, produced by Sumitomo 3M Ltd.) to have a thickness of 0.7 mm and be smoothened. The resulting bovine tooth disc was immersed in a solution prepared by diluting a 0.5 M EDTA solution (produced by Wako Pure Chemical Industries, Ltd.) five times, for 180 seconds and was washed in distilled water for about 30 seconds. It was further immersed in a 10% sodium hypochlorite solution (Neo-Cleaner "SEKINE" produced by Neo Dental Chemical Products Co., Ltd.) for 120 seconds and then was washed in distilled water for about 30 minutes, so that a bovine tooth disc to be used for dentin penetration inhibition ratio evaluation was prepared.

(2) Dentin penetration inhibition ratio evaluation test (initial) Preparation of samples

About 0.1 g of the dentinal tubule sealant prepared above was attached with a spatula to the cheek-side dentin surface of the above-described bovine tooth disc, and then it was rubbed to a dentin of 5 mm in diameter within the center portion of the treated dentin surface, for 30 seconds by using a microbrush ("REGULAR SIZE (2.0 mm), MRB400" produced by MICROBRUSH INTERNATIONAL) . Then, the paste on the dentin surface was removed with distilled water, and a dentin penetration inhibition ratio evaluation test (n = 5) was carried out immediately.

[Preparation of artificial saliva]

Sodium chloride (8.77 g, 150 mmol), potassium dihydrogen phosphate (122 mg, 0.9 mmol), calcium chloride (166 mg, 1.5 mmol), and Hepes (4.77 g, 20 mmol) were separately weighed out on weighing dishes and then added one after another to a 2000-ml beaker containing about 800 ml of distilled water. After confirmation of complete dissolution of the solutes, pH was adjusted to 7.0 by dropping a 10% aqueous sodium hydroxide solution while measuring the acidity of the solution with a pH meter (F55, manufactured by HORIBA, Ltd.). Subsequently, this solution was added to a 1000-ml volumetric flask and diluted, so that 1000 ml of artificial saliva was obtained.

(3) Dentin penetration inhibition ratio evaluation test (long term) Preparation of samples

About 0.1 g of the dentinal tubule sealant prepared above was attached with a spatula to the cheek-side dentin surface of the above-described bovine tooth disc, and then it was rubbed to a dentin of 5 mm in diameter within the center portion of the treated dentin surface, for 30 seconds by using a microbrush ("REGULAR SIZE (2.0 mm), MRB400" produced by MICROBRUSH INTERNATIONAL) . Then, the paste on the dentin surface was removed with distilled water, followed by immersion in artificial saliva for two weeks, and then a dentin penetration inhibition ratio evaluation test (n = 5) was carried out.

(4) Dentin penetration inhibition ratio evaluation test

Measurement of a dentin penetration inhibition ratio was performed using a method according to the method of Pashley et al. (D. H.PASHLEY et al., J. Dent. Res. 65:417-420, 1986; K. C. Y. TAY et al., J. Endod. 33:1438-1443, 2007). The same device was installed, and the bovine tooth disc having been subjected to the dentinal tubule sealing treatment was installed and fixed to a dividable chamber jig so that a liquid could penetrate in a direction from dental pulp toward enamel. The dentin surface to receive pressure of phosphate-buffered saline (Dulbecco's PBS, Grand Island Biological Company, Grand Island, NY) was standardized to a surface area of 78.5 $mm^2$ (5 mm in diameter) using an O ring and was pressurized at 10 psi (69 kPa), and then a penetrated amount was measured after a lapse of 24 hours. Moreover, a dentin penetration inhibition ratio was calculated using the following formula from the measurement of the penetrated amount of the same bovine tooth disc having not been subjected to the dentinal tubule sealing treatment by the same operation.

Dentin penetration inhibition ratio (%) = [1 - (penetrated amount

of a dentinal tubule-sealed bovine tooth disc)/(penetrated amount

of a dentinal tubule-unsealed bovine tooth disc)] × 100

[Poorly soluble calcium phosphate particle (A)]

DCPA: 10.3 $\mu$m, anhydrous calcium hydrogen phosphate [$CaHPO_4$], produced by Wako Pure Chemical Industries, Ltd.
DCPD: 5.1 $\mu$m, calcium hydrogen phosphate dihydrate [$CaHPO_4 \cdot 2H_2O$], produced by Taihei Chemical Industrial Co., Ltd.
$\beta$-TCP: 1.0 $\mu$m, $\beta$-tricalcium phosphate [$\beta$-$Ca_3(PO_4)_2$], produced by Taihei Chemical Industrial Co., Ltd.
OCP: 4. 8 $\mu$m, octacalcium phosphate pentahydrate [$Ca_8H_2(PO_4)_6 \cdot 5H_2O$] Ca pyrophosphate: 15.0 $\mu$m, calcium pyrophosphate [$Ca_2P_2O_7$], produced by Taihei Chemical Industrial Co., Ltd.

[Phosphorus-free calcium compound (B)]

Ca(OH)$_2$: 14.5 $\mu$m, calcium hydroxide, produced by KAWAI LIME INDUSTRY Co., Ltd.
CaO: 10.0 $\mu$m, calcium oxide, produced by Wako Pure Chemical Industries, Ltd.
Ca(NO$_3$)$_2$: calcium nitrate, produced by Wako Pure Chemical Industries, Ltd.
CaCl$_2$: calcium chloride, produced by Wako Pure Chemical Industries, Ltd.
CaSiO$_3$: calcium metasilicate, produced by Wako Pure Chemical Industries, Ltd.

[Alkali metal salt of phosphoric acid (D)]

Na$_2$HPO$_4$: disodium hydrogen phosphate, produced by Wako Pure Chemical Industries, Ltd.
NaH$_2$PO$_4$: sodium dihydrogen phosphate, produced by Wako Pure Chemical Industries, Ltd.

[Fluorine compound (E)]

NaF: sodium fluoride, produced by Wako Pure Chemical Industries, Ltd.
MFP: sodium monofluorophosphate, produced by Wako Pure Chemical Industries, Ltd.

[Silica particle (F)]

Ar130: "Aerosil 130 (commercial name) " produced by Nippon Aerosil Co., Ltd.

[Others]

HAp: 2.5 $\mu$m, hydroxyapatite (HAP-200), produced by Taihei Chemical Industrial Co., Ltd.
MCPA: 7.0 $\mu$m, monocalcium phosphate anhydrous, produced by Taihei Chemical Industrial Co., Ltd.

[Preparation of powders]

Preparation of DCPA: average particle diameter of 1.1 μm

DCPA having an average particle diameter of 1.1 μm was obtained by subjecting a slurry resulting from addition of 50 g of DCPA: 10.3 μm, 240 g of 95% ethanol ("Ethanol (95)" produced by Wako Pure Chemical Industries, Ltd.) and 480 g of zirconia balls having a diameter of 10 mm into a 1000-ml grinding pot made of alumina ("HD-B-104 Pot Mill" manufactured by Nikkato Corporation) and subsequent wet vibration pulverization at a rotation speed of 1500 rpm for 15 hours, to evaporation of ethanol with a rotary evaporator, followed by drying at 60°C for 6 hours. DCPA having an average particle diameter of 0.5 μm, that having an average particle diameter of 0.8 μm, that having an average particle diameter of 5.2 μm, and that having an average particle diameter of 7.5 μm were obtained in the same manner as described above using grinding times of 40 hours, 20 hours, 7 hours, and 3 hours, respectively.

Preparation of DCPD: average particle diameter of 1.1 μm

DCPD having an average particle diameter of 1.1 μm was obtained by subjecting a slurry resulting from addition of 50 g of DCPD: 5.1 μm, 240 g of 95% ethanol ("Ethanol (95)" produced by Wako Pure Chemical Industries, Ltd.) and 480 g of zirconia balls having a diameter of 10 mm into a 1000-ml grinding pot made of alumina ("HD-B-104 Pot Mill" manufactured by Nikkato Corporation) and subsequent wet vibration pulverization at a rotation speed of 1500 rpm for 10 hours, to evaporation of ethanol with a rotary evaporator, followed by drying at 60°C for 6 hours.

Preparation of OCP: 1.5 μm

A 0.04 M aqueous solution (250 ml) of calcium acetate (produced by Wako Pure Chemical Industries, Ltd.) and a 0.04 M aqueous $NaH_2PO_4$ solution (250 ml) were prepared. While the 0.04 M aqueous $NaH_2PO_4$ solution of 67.5°C was stirred with a magnetic stirrer at 400 rpm, the 0.04 M aqueous calcium acetate solution was dropped at 250 ml/hour, so that OCP crystals were obtained. The crystals obtained were vacuum dried at 60°C for 10 hours, affording crystals having a size of about 500 μm. OCP: 1.5 μm was obtained by subjecting a slurry resulting from addition of 50 g of the OCP obtained above, 240 g of 99.5% ethanol ("Ethanol, Dehydrated (99.5)" produced by Wako Pure Chemical Industries, Ltd.) and 480 g of zirconia balls having a diameter of 10 mm into a 1000-ml grinding pot made of alumina ("HD-B-104 Pot Mill" manufactured by Nikkato Corporation) and subsequent wet vibration pulverization at a rotation speed of 1500 rpm for 15 hours, to evaporation of ethanol with a rotary evaporator, followed by vacuum drying at 60°C for 6 hours.

Preparation of Ca pyrophosphate: 0.9 μm

Ca pyrophosphate having an average particle diameter of 0.9 μm was obtained by subjecting a slurry resulting from addition of 50 g of Ca pyrophosphate: 15.0 μm, 240 g of 99.5% ethanol ("Ethanol, Dehydrated (99.5)" produced by Wako Pure Chemical Industries, Ltd.) and 480 g of zirconia balls having a diameter of 10 mm into a 1000-ml grinding pot made of alumina ("HD-B-104 Pot Mill" manufactured by Nikkato Corporation) and subsequent wet vibration pulverization at a rotation speed of 1500 rpm for 15 hours, to evaporation of ethanol with a rotary evaporator, followed by vacuum drying at 60°C for 6 hours.

Preparation of $Ca(OH)_2$: average particle diameter of 1.0 μm

$Ca(OH)_2$ having an average particle diameter of 1.0 μm was obtained by subjecting a slurry resulting from addition of 50 g of $Ca(OH)_2$: 14.5 μm, 240 g of 99.5% ethanol ("Ethanol, Dehydrated (99.5) " produced by Wako Pure Chemical Industries, Ltd.) and 480 g of zirconia balls having a diameter of 10 mm into a 1000-ml grinding pot made of alumina ("HD-B-104 Pot Mill" manufactured by Nikkato Corporation) and subsequent wet vibration pulverization at a rotation speed of 1500 rpm for 15 hours, to evaporation of ethanol with a rotary evaporator, followed by drying at 60°C for 6 hours. $Ca(OH)_2$ having an average particle diameter of 0.5 μm, that having an average particle diameter of 5.2 μm, and that having an average particle diameter of 10.0 μm were obtained in the same manner as described above using grinding times of 20 hours, 7 hours, and 3 hours, respectively.

Preparation of $Ca(NO_3)_2$: 5.0 μm

$Ca(NO_3)_2$ having an average particle diameter of 5.0 μm was obtained by subjecting a slurry resulting from addition of 50 g of $Ca(NO_3)_2$, 240 g of 99.5% ethanol ("Ethanol, Dehydrated (99.5)" produced by Wako Pure Chemical Industries, Ltd.) and 480 g of zirconia balls having a diameter of 10 mm into a 1000-ml grinding pot made of alumina ("HD-B-104 Pot Mill" manufactured by Nikkato Corporation) and subsequent wet vibration pulverization at a rotation speed of 1500 rpm for 10 hours, to evaporation of ethanol with a rotary evaporator, followed by vacuum drying at 60°C for 6 hours.

Preparation of $CaCl_2$: 5.0 $\mu$m

$CaCl_2$ having an average particle diameter of 5.0 $\mu$m was obtained by subjecting a slurry resulting from addition of 50 g of $CaCl_2$, 240 g of 99.5% ethanol ("Ethanol, Dehydrated (99.5)" produced by Wako Pure Chemical Industries, Ltd.) and 480 g of zirconia balls having a diameter of 10 mm into a 1000-ml grinding pot made of alumina ("HD-B-104 Pot Mill" manufactured by Nikkato Corporation) and subsequent wet vibration pulverization at a rotation speed of 1500 rpm for 10 hours, to evaporation of ethanol with a rotary evaporator, followed by vacuum drying at 60°C for 6 hours.

Preparation of $CaSiO_3$: 5.0 $\mu$m

$CaSiO_3$ having an average particle diameter of 5.0 $\mu$m was obtained by subjecting a slurry resulting from addition of 50 g of $CaSiO_3$, 240 g of 99.5% ethanol ("Ethanol, Dehydrated (99.5)" produced by Wako Pure Chemical Industries, Ltd.) and 480 g of zirconia balls having a diameter of 10 mm into a 1000-ml grinding pot made of alumina ("HD-B-104 Pot Mill" manufactured by Nikkato Corporation) and subsequent wet vibration pulverization at a rotation speed of 1500 rpm for 15 hours, to evaporation of ethanol with a rotary evaporator, followed by vacuum drying at 60°C for 6 hours.

Preparation of CaO: 5.0 $\mu$m

CaO having an average particle diameter of 2.0 $\mu$m was obtained by subjecting a slurry resulting from addition of 50 g of CaO: 10.0 $\mu$m, 240 g of 99.5% ethanol ("Ethanol, Dehydrated (99.5)" produced by Wako Pure Chemical Industries, Ltd.) and 480 g of zirconia balls having a diameter of 10 mm into a 1000-ml grinding pot made of alumina ("HD-B-104 Pot Mill" manufactured by Nikkato Corporation) and subsequent wet vibration pulverization at a rotation speed of 1500 rpm for 10 hours, to evaporation of ethanol with a rotary evaporator, followed by vacuum drying at 60°C for 6 hours.

Preparation of $Na_2HPO_4$: 4.6 $\mu$m

$Na_2HPO_4$ having an average particle diameter of 4.6 $\mu$m was prepared by once treating $Na_2HPO_4$ with a Nanojetmizer (Model NJ-100 manufactured by Aishin Nano Technologies Co., Ltd.) while adjusting grinding pressure condition to feeding pressure of 0.7 MPa/grinding pressure of 0.7 MPa, and treated amount condition to 8 kg/hr.

Preparation of $Na_2HPO_4$: 9.7 $\mu$m

$Na_2HPO_4$ having an average particle diameter of 9.7 $\mu$m was prepared by once treating $Na_2HPO_4$ with a Nanojetmizer (Model NJ-100 manufactured by Aishin Nano Technologies Co., Ltd.) while adjusting grinding pressure condition to feeding pressure, 0.3 MPa/grinding pressure, 0.3 MPa and treated amount condition to 8 kg/hr.

Preparation of $Na_2HPO_4$: 19.7 $\mu$m

$Na_2HPO_4$ having an average particle diameter of 19.7 $\mu$m was prepared by once treating $Na_2HPO_4$ with a Nanojetmizer (Model NJ-100 manufactured by Aishin Nano Technologies Co., Ltd.) while adjusting grinding pressure condition to feeding pressure, 0.2 MPa/grinding pressure, 0.1 MPa and treated amount condition to 20 kg/hr.

Preparation of $Na_2HPO_4$: 1.45 $\mu$m

$Na_2HPO_4$ having an average particle diameter of 1.45 $\mu$m was prepared by four times treating $Na_2HPO_4$ with a Nanojetmizer (Model NJ-100 manufactured by Aishin Nano Technologies Co., Ltd.) while adjusting grinding pressure condition to feeding pressure, 1.3 MPa/grinding pressure, 1.3 MPa and treated amount condition to 1 kg/hr.

Preparation of $Na_2HPO_4$: 0.65 $\mu$m

$Na_2HPO_4$ having an average particle diameter of 0.65 $\mu$m was prepared by five times treating $Na_2HPO_4$: 1.45

µm with a Nanojetmizer (Model NJ-100 manufactured by Aishin Nano Technologies Co., Ltd.) while adjusting grinding pressure condition to feeding pressure, 1.3 MPa/grinding pressure, 1.3 MPa and treated amount condition to 1 kg/hr.

Preparation of $NaH_2PO_4$: 4.8 µm

$NaH_2PO_4$ having an average particle diameter of 4.8 µm was prepared by once treating $NaH_2PO_4$ with a Nanojetmizer (Model NJ-100 manufactured by Aishin Nano Technologies Co., Ltd.) while adjusting grinding pressure condition to feeding pressure, 0.7 MPa/grinding pressure, 0.7 MPa and treated amount condition to 8 kg/hr.

[Preparation of dentinal tubule sealant]

(1) Preparation of powder for dentinal tubule sealant

A powder of a dentinal tubule sealant was prepared by adding powder components weighed in the composition given in Table 1 to a high-speed rotation mill (AS ONE Corporation "SM-1") and mixing them at a rotation speed of 1000 rpm for 3 minutes. A powder not needing mixing was used as it was as a powder of a dentinal tubule sealant.

(2) Preparation of liquid agent for dentinal tubule sealant

A liquid agent for a dentinal tubule sealant was obtained by dissolving liquid agent components weighed in the composition given in Table 1 and 2 in distilled water. In the case of a composition containing no liquid agent components, distilled water was used as it was as a liquid agent for a dentinal tubule sealant.

(3) Preparation of dentinal tubule sealant

Dentinal tubule sealants were prepared by adding and mixing the powders described in (1) above with compositions given in Tables 1 and 2 and the liquid agents obtained in (2) above.

Examples 1 to 45

Dentinal tubule sealants were prepared in the above-described procedures (1) to (3) and then an initial dentin penetration inhibition ratio evaluation test and a long-term dentin penetration inhibition ratio evaluation test were performed. The evaluation results obtained are summarized in Tables 1 and 2. The examples 4, 5, 9 and 12 depicted in Table 1 are not part of the present invention.

(1) Production of bovine tooth for morphological evaluation

A cheek-side center of a healthy bovine incisor tooth was trimmed with #80, #1000 sand papers by using a rotary grinder, so that a 2 mm thick dentin plate with a cheek-side dentin exposed was produced. This cheek-side dentin surface was further polished with wrapping films (#1200, #3000, #8000, produced by Sumitomo 3M Ltd.) to be smoothened. This cheek-side dentin portion was masked with manicure with a window of a test portion as large as 7 mm in both the ordinate direction and the abscissa direction left unmasked, and then was air-dried for one hour. As to this bovine tooth, a solution prepared by diluting a 0.5-M EDTA solution (produced by Wako Pharmaceutical) five times was applied to the dentin window for 30 seconds to perform demineralization, followed by washing with water for 30 minutes or more. Moreover, it was cleaned by applying a 10% sodium hypochlorite solution (Neo-Cleaner "SEKINE" produced by Neo Dental Chemical Products Co., Ltd.) to it for two minutes and then was washed in water for about 30 minutes or more, so that a bovine tooth to be used for dentinal tubule sealing evaluation was prepared. After the above-described tooth surface treatment, half of the tooth surface along the ordinate direction of the tooth was masked with manicure, so that its untreated state was maintained. About 0.1 g of the dentinal hypersensitivity inhibitor of Example 8 was attached with a spatula to the cheek-side dentin surface of the above-described bovine tooth, and then it was rubbed to the entire dentin window for 30 seconds by using a microbrush ("REGULAR SIZE (2.0 mm), MRB400" produced by MICROBRUSH INTERNATIONAL). Then, the paste on the dentin surface was removed with distilled water.

(2) Production of sample for SEM observation

After the above-described treatment, the bovine tooth sample was immersed in a 70% aqueous ethanol solution in a vial. Immediately after the immersion, the vial was moved into a desiccator and was placed under a reduced pressure condition for 10 minutes. Then, the vial was taken out from the desiccator and it was attached to a low-speed stirrer (TR-118, manufactured by AS ONE Corporation), followed by stirring at a rotation speed of about 4 rpm for 1 hour. The same operations were performed using a 80% aqueous ethanol solution, a 90% aqueous ethanol solution, a 99% aqueous ethanol solution, and 100% ethanol (twice), wherein the bovine tooth was immersed in the second 100% ethanol continuously for one night. Next day, the same operations were carried out sequentially for a 1:1 mixed solvent of propylene oxide and ethanol and for 100% propylene oxide (twice), wherein the bovine tooth was immersed in the second propylene oxide continuously for one night, so that dehydration and removal of the manicure were performed. The sample from which propylene oxide had been evaporated away was determined as a sample for morphological observation of a dentinal tubule sealing-treated surface of the bovine tooth disc. Moreover, after the evaporation of propylene oxide, the dentinal tubule sealing-treated dentin was fractured brittly by using two pliers, thereby obtaining a sample for morphological observation of a cross section of the dentin.

(3) SEM observation

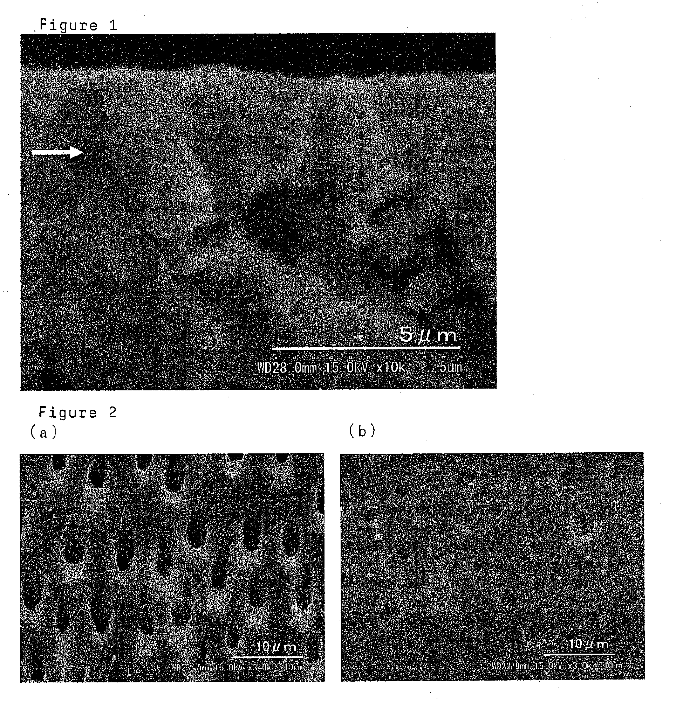

For SEM observation was used an S-3500N (manufactured by Hitachi High-Technologies Corporation) . The surface morphology in the vicinity of a boundary between a dentinal tubule sealing-treated portion and an untreated portion of a bovine tooth disc before fracture and the morphology in the vicinity of a dentinal tubule sealing-treated surface of a cross section of the dentin were observed at an accelerating voltage of 15 kV, and a deepest distance from a mineralized dentin surface at which distance closure by a hypersensitivity inhibitor could be observed in the dentinal tubule direction (hereinafter sometimes referred to also as a "dentinal tubule sealing depth") was measured. The average of the dentinal tubule sealing depth by the hypersensitivity inhibitor of Example 8 was 10 $\mu$m. SEM photographs obtained are shown in Fig. 1 and Fig. 2 (the item pointed by the arrow in Fig. 1 is a dentinal tubule sealed with HAp).

Comparative Examples 1 to 6

Dentinal tubule sealants were prepared in the above-described procedures (1) to (3) and then an initial dentin penetration inhibition ratio evaluation test and a long term dentin penetration inhibition ratio evaluation test were performed. The evaluation results obtained are summarized in Table 3.

Example 46

A nonaqueous paste was prepared by mixing 20.5 g of DCPA: 1.1 $\mu$m, 0.5 g of Ca(OH)$_2$: 5.2 $\mu$m, 4 g of Na$_2$HPO$_4$: 4.6 $\mu$m, 0.22 g of NaF, 0.5 g of Ar130, and 13.78 g of glycerol (produced by Wako Pure Chemical Industries, Ltd.). An aqueous paste was prepared by mixing 20.0 g of DCPA: 1.1 $\mu$m, 0.5 g of sodium saccharate (produced by Wako Pure Chemical Industries, Ltd.), 3 g of polyethylene glycol (Macrogol 400, produced by Sanyo Chemical Industries, Ltd.), 5 g of glycerol , 5.0 g of propylene glycol (produced by Wako Pure Chemical Industries, Ltd.) , 0.05 g of cetyl pyridinium chloride monohydrate (produced by Wako Pure Chemical Industries, Ltd.), 3.5 g of Ar130, and 23.45 g of distilled water. A dentinal tubule sealant was prepared by adding 39.5 g of the nonaqueous paste prepared above and 60.5 g of the aqueous paste prepared above, and then mixing them. In the same manner as in Example 1, an initial dentin penetration inhibition ratio evaluation test and a long term dentin penetration inhibition ratio evaluation test were performed. The evaluation results obtained are summarized in Table 4.

Examples 47 to 49

Dentinal tubule sealants were prepared in the same manner as in Example 46 and then an initial dentin penetration inhibition ratio evaluation test and a long term dentin penetration inhibition ratio evaluation test were performed. The evaluation results obtained are summarized in Table 3.

Example 50

A nonaqueous paste 1 was prepared by mixing 40.5 g of DCPA: 1.1 $\mu$m, 4 g of Na$_2$HPO$_4$: 4.6 $\mu$m, 0.22 g of NaF, 0.5 g of Ar130, 13.78 g of glycerol (produced by Wako Pure Chemical Industries, Ltd.), and 23.0 g of distilled water. An aqueous paste 2 was prepared by mixing 0.5 g of Ca(OH)$_2$: 5.2 $\mu$m, 0.5 g of sodium saccharate (produced by Wako Pure Chemical Industries, Ltd.), 3 g of polyethylene glycol (Macrogol 400, produced by Sanyo Chemical Industries, Ltd.), 5.0 g of propylene glycol (produced by Wako Pure Chemical Industries, Ltd.), 0.05 g of cetyl pyridinium chloride monohydrate (produced by Wako Pure Chemical Industries, Ltd.), 3.5 g of Ar130, and 5.45 g of distilled water. A dentinal tubule sealant was prepared by adding 82.0 g of the aqueous paste 1 prepared above and 18.0 g of the aqueous paste 2 prepared above, and then mixing them. In the same manner as in Example 1, an initial dentin penetration inhibition ratio evaluation test and a long-term dentin penetration inhibition ratio evaluation test were performed. The evaluation results obtained are summarized in Table 5.

[Table 1]

| | | | Example 1 | Example 2 | Example 3 | Example 4 | Example 5 | Example 6 | Example 7 | Example 8 | Example 9 | Example 10 | Example 11 | Example 12 | Example 13 | Example 14 | Example 15 | Example 16 | Example 17 | Example 18 | Example 19 | Example 20 | Example 21 |
|---|---|---|---|---|---|---|---|---|---|---|---|---|---|---|---|---|---|---|---|---|---|---|---|
| Powder agent | A | DCPA: 0.5μm (% by weight) | | | | | | | | | | | | | | | | | | | | | |
| | | DCPA: 0.8μm (% by weight) | | | | | | | | | | | | | | | | | | | | | 55 |
| | | DCPA: 1.1μm (% by weight) | 31 | 75 | 55 | 55 | 55 | 55 | 55 | 55 | 35 | 45 | 65 | 30 | 55 | 50.5 | 55 | 55 | 55 | 55 | 55 | 55 | |
| | | DCPA: 5.2μm (% by weight) | | | | | | | | | | | | | | | | | | | | | |
| | | DCPA: 7.5μm (% by weight) | | | | | | | | | | | | | | | | | | | | | |
| | | DCPA: 10.3μm (% by weight) | | | | | | | | | | | | | | | | | | | | | |
| | | DCPD: 1.1μm (% by weight) | | | | | | | | | | | | | | | | | | | | | |
| | | β-TCP: 1.0μm (% by weight) | | | | | | | | | | | | | | | | | | | | | |
| | | OCP: 1.5μm (% by weight) | | | | | | | | | | | | | | | | | | | | | |
| | | Ca pyrophosphate: 0.9μm (% by weight) | | | | | | | | | | | | | | | | | | | | | |
| | B | Ca(OH)₂: 0.5μm (% by weight) | | | | | | | | | | | | | | | | | | | | | |
| | | Ca(OH)₂: 1.0μm (% by weight) | | | | | | | | | | | | | | | | | | | | | |
| | | Ca(OH)₂: 5.2μm (% by weight) | | | 0.1 | 4 | | | | 0.5 | 3.5 | 1.5 | 0.5 | 4 | 0.5 | 0.5 | 0.5 | 0.5 | 0.5 | 0.5 | 0.5 | 0.5 | 0.5 |
| | | Ca(OH)₂: 10.0μm (% by weight) | | | | | | | | | | | | | | | | | | | | | |
| | | Ca(OH)₂: 14.5μm (% by weight) | | | | | | | | | | | | | | | | | | | | | |
| | | CaO: 5.0μm (% by weight) | | | | | | | | | | | | | | | | | | | | | |
| | | Ca(NO₃)₂: 5.0μm (% by weight) | | | | | | | | | | | | | | | | | | | | | |
| | | CaCl₂: 5.0μm (% by weight) | | | | | | | | | | | | | | | | | | | | | |
| | | CaSiO₃: 5.0μm (% by weight) | | | | | | | | | | | | | | | | | | | | | |
| | D | Na₂HPO₄: 0.65μm (% by weight) | | | | | | | | | | | | | | | | | | | | | |
| | | Na₂HPO₄: 1.45μm (% by weight) | | | | | | | | | | | | | | | | | | | | | |
| | | Na₂HPO₄: 4.6μm (% by weight) | | | | | | | | | | | | | 0.5 | 25 | | | 10 | 10 | 10 | 10 | 5 |
| | | Na₂HPO₄: 9.7μm (% by weight) | | | | | | | | | | | | | | | | | | | | | |
| | | Na₂HPO₄: 19.7μm (% by weight) | | | | | | | | | | | | | | | | | | | | | |
| | | NaH₂PO₄: 4.8μm (% by weight) | | | | | | | | | | | | | | | | | | | | | |
| | E | NaF (% by weight) | | | | | | | | | | | | | | | | | | | | | |
| | F | Ar130 (% by weight) | | | | | | | | | | | | | | | | | | | | 0.5 | 3 |
| Amount of powder agent (% by weight) | | | 31.0 | 75.0 | 55.1 | 59.0 | 55.0 | 55.0 | 55.0 | 55.5 | 38.5 | 46.5 | 65.5 | 34.0 | 56.0 | 76.0 | 55.5 | 55.5 | 65.5 | 65.5 | 66.0 | 68.5 | 60.5 |
| Liquid agent | B | Ca(OH)₂ (% by weight) | 0.05 | 0.05 | | | 0.001 | 0.1 | 0.05 | | | | | | | | | | | | | | |
| | | Ca(NO₃)₂ (% by weight) | | | | | | | | | | | | | | | | | | | | | |
| | | CaCl₂ (% by weight) | | | | | | | | | | | | | | | | | | | | | |
| | D | Na₂HPO₄ (% by weight) | | | | | | | | | | | | | | | 0.5 | 2 | | | | | |
| | E | NaF (% by weight) | | | | | | | | 0.22 | 0.22 | 0.22 | 0.22 | 0.22 | 0.22 | 0.22 | 0.22 | 0.22 | 0.01 | 3 | 0.22 | 0.22 | 0.22 |
| | | MFP (% by weight) | | | | | | | | | | | | | | | | | | | | | |
| | C | Purified water (% by weight) | 68.95 | 24.95 | 44.9 | 41 | 45 | 44.9 | 44.73 | 44.28 | 61.28 | 53.28 | 34.28 | 65.78 | 43.78 | 23.78 | 43.78 | 42.28 | 34.49 | 31.5 | 33.78 | 31.28 | 39.28 |
| Amount of liquid agent (% by weight) | | | 69 | 25 | 44.9 | 41 | 45 | 45 | 45 | 44.5 | 61.5 | 53.5 | 34.5 | 66 | 44 | 24 | 44.5 | 44.5 | 34.5 | 34.5 | 34 | 31.5 | 39.5 |
| Total (% by weight) | | | 100 | 100 | 100 | 100 | 100 | 100 | 100 | 100 | 100 | 100 | 100 | 100 | 100 | 100 | 100 | 100 | 100 | 100 | 100 | 100 | 100 |
| Dentin penetration inhibition ratio (initial) (%) | | | 43 | 42 | 67 | 40 | 37 | 73 | 83 | 90 | 48 | 53 | 58 | 35 | 92 | 43 | 90 | 89 | 85 | 87 | 86 | 87 | 69 |
| Dentin penetration inhibition ratio (long term) (%) | | | 56 | 64 | 87 | 48 | 63 | 84 | 86 | 92 | 60 | 63 | 72 | 37 | 94 | 50 | 92 | 90 | 88 | 91 | 90 | 91 | 64 |
| Ca/P | | | 1.003 | 1.001 | 1.003 | 1.133 | 1.000 | 1.003 | 1.002 | 1.017 | 1.184 | 1.061 | 1.014 | 1.245 | 1.017 | 1.018 | 1.017 | 1.017 | 1.017 | 1.017 | 1.017 | 1.017 | 1.017 |
| P/L | | | 0.449 | 3.000 | 1.227 | 1.439 | 1.222 | 1.222 | 1.222 | 1.247 | 0.626 | 0.869 | 1.899 | 0.515 | 1.273 | 3.167 | 1.247 | 1.247 | 1.899 | 1.899 | 1.941 | 2.175 | 1.532 |

20

[Table 2]

| Powder agent | Component | Example 22 | Example 23 | Example 24 | Example 25 | Example 26 | Example 27 | Example 28 | Example 29 | Example 30 | Example 31 | Example 32 | Example 33 | Example 34 | Example 35 | Example 36 | Example 37 | Example 38 | Example 39 | Example 40 | Example 41 | Example 42 | Example 43 | Example 44 | Example 45 |
|---|---|---|---|---|---|---|---|---|---|---|---|---|---|---|---|---|---|---|---|---|---|---|---|---|---|
| A | DCPA: 0.5μm (% by weight) | | | | | | | | | | | | | | | | | | | | | | | | |
| | DCPA: 0.8μm (% by weight) | | 55 | | | | | | | | | | | | | | | | | | | | | | |
| | DCPA: 1.1μm (% by weight) | | | | | 55 | 55 | 55 | 55 | 55 | 55 | 55 | 55 | | | | | 55 | 55 | 55 | 55 | 55 | 55 | 55 | 55 |
| | DCPA: 5.2μm (% by weight) | | | 55 | | | | | | | | | | | | | | | | | | | | | |
| | DCPA: 7.5μm (% by weight) | | | | 55 | | | | | | | | | | | | | | | | | | | | |
| | DCPA: 10.3μm (% by weight) | 55 | | | | | | | | | | | | | | | | | | | | | | | |
| | DCPD: 1.1μm (% by weight) | | | | | | | | | | | | | 55 | | | | | | | | | | | |
| | β-TCP: 1.0μm (% by weight) | | | | | | | | | | | | | | 55 | | | | | | | | | | |
| | OCP: 1.5μm (% by weight) | | | | | | | | | | | | | | | 55 | | | | | | | | | |
| | Ca pyrophosphate: 0.9μm (% by weight) | | | | | | | | | | | | | | | | 55 | | | | | | | | |
| B | Ca(OH)₂: 0.5μm (% by weight) | | | | | | | 0.5 | | | | | | | | | | | | | | | | | |
| | Ca(OH)₂: 1.0μm (% by weight) | | | | | 0.5 | | | | | | | | | | | | | | | | | | | |
| | Ca(OH)₂: 5.2μm (% by weight) | 0.5 | 0.5 | 0.5 | 0.5 | | | | | 0.5 | 0.5 | 0.5 | 0.5 | 0.5 | 0.5 | 0.5 | 0.5 | | | | | | | 0.5 | 0.5 |
| | Ca(OH)₂: 10.0μm (% by weight) | | | | | | 0.5 | | | | | | | | | | | | | | | | | | |
| | Ca(OH)₂: 14.5μm (% by weight) | | | | | | | | 0.5 | | | | | | | | | | | | | | | | |
| | CaO: 5.0μm (% by weight) | | | | | | | | | | | | | | | | | 0.5 | | | | | | | |
| | Ca(NO₃)₂: 5.0μm (% by weight) | | | | | | | | | | | | | | | | | | 0.5 | | | | | | |
| | CaCl₂: 5.0μm (% by weight) | | | | | | | | | | | | | | | | | | | 0.5 | | | | | |
| | CaSiO₃: 5.0μm (% by weight) | | | | | | | | | | | | | | | | | | | | 0.5 | | | | |
| D | Na₂HPO₄: 0.65μm (% by weight) | | | | | | | | | | | 5 | | | | | | | | | | | | | |
| | Na₂HPO₄: 1.45μm (% by weight) | | | | | | | | | | 5 | | | | | | | | | | | | | | |
| | Na₂HPO₄: 4.6μm (% by weight) | 5 | 5 | 5 | 5 | 5 | 5 | 5 | 5 | | | | | 5 | 5 | 5 | 5 | 5 | 5 | 5 | 5 | 5 | 5 | 5 | |
| | Na₂HPO₄: 9.7μm (% by weight) | | | | | | | | | 5 | | | | | | | | | | | | | | | |
| | Na₂HPO₄: 19.7μm (% by weight) | | | | | | | | | | | | 5 | | | | | | | | | | | | |
| | NaH₂PO₄: 4.8μm (% by weight) | | | | | | | | | | | | | | | | | | | | | | | | 5 |
| E | NaF (% by weight) | | 0.22 | 0.22 | 0.22 | | | | | | | | | | | | | | | | | | | | |
| F | Ar130 (% by weight) | | | | | | | | | | | | | | | | | | | | | | | | |

EP 2 626 058 B1

EP 2 626 058 B1

[Table 2] (continued)

| | | (unit) | Example 22 | Example 23 | Example 24 | Example 25 | Example 26 | Example 27 | Example 28 | Example 29 | Example 30 | Example 31 | Example 32 | Example 33 | Example 34 | Example 35 | Example 36 | Example 37 | Example 38 | Example 39 | Example 40 | Example 41 | Example 42 | Example 43 | Example 44 | Example 45 |
|---|---|---|---|---|---|---|---|---|---|---|---|---|---|---|---|---|---|---|---|---|---|---|---|---|---|---|
| Amount of powder agent | | (% by weight) | 60.5 | 60.7 | 60.7 | 60.7 | 60.5 | 60.5 | 60.5 | 60.5 | 60.5 | 60.5 | 60.5 | 60.5 | 60.5 | 60.5 | 60.5 | 60.5 | 60.5 | 60.5 | 60.5 | 60.5 | 60.0 | 60.0 | 60.5 | 60.5 |
| Liquid agent | B | $Ca(OH)_2$ (% by weight) | | | | | | | | | | | | | | | | | | | | | | | | |
| | B | $Ca(NO_3)_2$ (% by weight) | | | | | | | | | | | | | | | | | | | | | 0.05 | | | |
| | | $CaCl_2$ (% by weight) | | | | | | | | | | | | | | | | | | | | | | 0.05 | | |
| | C | $Na_2HPO_4$ (% by weight) | | | | | | | | | | | | | | | | | | | | | | | | |
| | E | NaF (% by weight) | 0.22 | | | | 0.22 | 0.22 | 0.22 | 0.22 | 0.22 | 0.22 | 0.22 | 0.22 | 0.22 | 0.22 | 0.22 | 0.22 | 0.22 | 0.22 | 0.22 | 0.22 | 0.22 | 0.22 | | 0.22 |
| | | MFP (% by weight) | | | | | | | | | | | | | | | | | | | | | | | 0.22 | |
| | C | Purified water (% by weight) | 39.28 | 39.28 | 39.28 | 39.28 | 39.28 | 39.28 | 39.28 | 39.28 | 39.28 | 39.28 | 39.28 | 39.28 | 39.28 | 39.28 | 39.28 | 39.28 | 39.28 | 39.28 | 39.28 | 39.28 | 39.73 | 39.73 | 39.28 | 39.28 |
| Amount of liquid agent | | (% by weight) | 39.5 | 39.28 | 39.28 | 39.28 | 39.5 | 39.5 | 39.5 | 39.5 | 39.5 | 39.5 | 39.5 | 39.5 | 39.5 | 39.5 | 39.5 | 39.5 | 39.5 | 39.5 | 39.5 | 39.5 | 40 | 40 | 39.5 | 39.5 |
| Total | | (% by weight) | 100 | 100 | 100 | 100 | 100 | 100 | 100 | 100 | 100 | 100 | 100 | 100 | 100 | 100 | 100 | 100 | 100 | 100 | 100 | 100 | 100 | 100 | 100 | 100 |
| Dentin penetration inhibition ratio (initial) | | (%) | 51 | 89 | 62 | 58 | 87 | 83 | 73 | 40 | 72 | 70 | 33 | 62 | 91 | 87 | 81 | 84 | 90 | 75 | 70 | 70 | 77 | 72 | 88 | 91 |
| Dentin penetration inhibition ratio (long term) | | (%) | 53 | 90 | 67 | 60 | 90 | 90 | 76 | 45 | 78 | 75 | 33 | 70 | 95 | 90 | 83 | 88 | 94 | 83 | 77 | 77 | 83 | 78 | 95 | 94 |
| Ca/P | | | | 1.017 | 1.017 | 1.017 | 1.017 | 1.017 | 1.017 | 1.017 | 1.017 | 1.017 | 1.017 | 1.017 | 1.017 | 1.038 | 1.017 | 1.017 | 1.022 | 1.017 | 1.017 | 1.017 | 1.000 | 1.000 | 1.017 | 1.017 |
| P/L | | | 1.532 | 1.546 | 1.546 | 1.546 | 1.532 | 1.532 | 1.532 | 1.532 | 1.532 | 1.532 | 1.532 | 1.532 | 1.532 | 1.532 | 1.532 | 1.532 | 1.532 | 1.532 | 1.532 | 1.532 | 1.500 | 1.500 | 1.532 | 1.532 |

[Table 3]

| | | | | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|---|
| Powder agent | A | DCPA: 5.0μm | (% by weigh) | 25 | 80 | 55 | 55 | 40 | | |
| | B | Ca(OH)$_2$: 5.2μm | (% by weigh) | 0.5 | 0.5 | 0 | 7.5 | 15 | 0.5 | 0.5 |
| | | HAp: 2.5μm | (% by weigh) | | | | | | 55 | |
| | | MCPA: 7.0μm | (% by weigh) | | | | | | | 55 |
| Powder agent | | | (% by weigh) | 25.5 | 80.5 | 55 | 62.5 | 55 | 55.5 | 55.5 |
| Liquid agent | C | Purified water | (% by weigh) | 74.5 | 19.5 | 45 | 37.5 | 45 | 44.5 | 44.5 |
| Total | | | (% by weigh) | 100 | 100 | 100 | 100 | 100 | 100 | 100 |
| Dentin penetration inhibition ratio (initial) | | | (%) | 19 | 21 | 30 | 32 | 29 | 30 | 6 |
| Dentin penetration inhibition ratio (long term) | | | (%) | 28 | 29 | 24 | 35 | 27 | 12 | 8 |
| Ca/P | | | | 1.037 | 1.011 | 1.000 | 1.250 | 1.688 | 1.687 | 0.514 |
| P/L | | | | 0.342 | 4.128 | 1.222 | 1.667 | 1.222 | 1.247 | 1.247 |

[Table 4]

| | | | | Example 46 | Example 47 | Example 48 | Example 49 |
|---|---|---|---|---|---|---|---|
| Powder agent | A | DCPA: 1.1μm | (% by weigh) | 0 | 40.5 | 0 | 0 |
| | B | Ca(OH)$_2$: 5.2μm | (% by weigh) | 0 | 0.5 | 0 | 0 |
| | D | Na$_2$HPO$_4$: 4.6μm | (% by weigh) | 0 | 4 | 0 | 0 |
| Powder agent | | | (% by weigh) | 0.0 | 45.0 | 0.0 | 0.0 |
| Nonaqueous paste | A | DCPA: 1.1μm | (% by weigh) | 20.5 | 0 | 0 | 40.5 |
| | B | Ca(OH)$_2$: 5.2μm | (% by weigh) | 0.5 | 0 | 0.5 | 0 |
| | D | Na$_2$HPO$_4$: 4.6μm | (% by weigh) | 4 | 0 | 4 | 4 |
| | E | NaF | (% by weigh) | 0.22 | 0 | 0.22 | 0.22 |
| | F | Ar130 | (% by weigh) | 0.5 | 0 | 0.5 | 0.5 |
| | | Glycerol | (% by weigh) | 13.78 | 0 | 13.78 | 13.78 |
| Nonaqueous paste | | | (% by weigh) | 39.50 | 0.00 | 19.00 | 59.00 |
| Aqueous paste | A | DCPA: 1.1μm | (% by weigh) | 20 | 0 | 40.5 | 0 |
| | B | Ca(OH)$_2$: 5.2μm | (% by weigh) | 0 | 0 | 0 | 0.5 |
| | E | NaF | (% by weigh) | 0 | 0.22 | 0 | 0 |
| | | Sodium saccharate | (% by weigh) | 0.5 | 0.5 | 0.5 | 0.5 |
| | | Polyethylene glycol | (% by weigh) | 3 | 3 | 3 | 3 |
| | | Glycerol | (% by weigh) | 5 | 18.78 | 5 | 5 |
| | | Propylene glycol | (% by weigh) | 5 | 5 | 5 | 5 |
| | | Cetyl pyridinium chloride | (% by weigh) | 0.05 | 0.05 | 0.05 | 0.05 |
| | F | Ar130 | (% by weigh) | 3.5 | 4 | 3.5 | 3.5 |
| | C | Purified water | (% by weigh) | 23.45 | 23.45 | 23.45 | 23.45 |
| Aqueous paste | | | (% by weigh) | 60.50 | 55.00 | 81.00 | 41.00 |
| Total | | | (% by weigh) | 100.0 | 100.0 | 100.0 | 100.0 |
| Dentin penetration inhibition ratio (initial) | | | (%) | 83 | 82 | 80 | 83 |
| Dentin penetration inhibition ratio (long term) | | | (%) | 87 | 85 | 87 | 84 |
| Ca/P | | | | 1.023 | 1.023 | 1.023 | 1.023 |
| P/L | | | | 1.469 | 0.818 | 1.469 | 1.439 |

[Table 5]

| | | | | Example 50 |
|---|---|---|---|---|
| Aqueous paste 1 | A | DCPA: 1.1μm | (% by weigh) | 40.5 |
| | D | Na$_2$HPO$_4$: 4.6μm | (% by weigh) | 4 |
| | E | NaF | (% by weigh) | 0.22 |
| | F | Ar130 | (% by weigh) | 0.5 |
| | | Glycerol | (% by weigh) | 13.78 |
| | C | Purified water | (% by weigh) | 23 |
| Aqueous paste 1 | | | (% by weigh) | 82.00 |
| Aqueous paste 2 | B | Ca(OH)$_2$: 5.2μm | (% by weigh) | 0.5 |
| | | Sodium saccharate | (% by weigh) | 0.5 |
| | | Polyethylene glycol | (% by weigh) | 3 |
| | | Propylene glycol | (% by weigh) | 5 |
| | | Cetyl pyridinium chloride | (% by weigh) | 0.05 |
| | F | Ar130 | (% by weigh) | 3.5 |
| | C | Purified water | (% by weigh) | 5.45 |
| Aqueous paste 2 | | | (% by weigh) | 18.00 |
| Total | | | (% by weigh) | 100.00 |
| Dentin penetration inhibition ratio (initial) | | | (%) | 85 |
| Dentin penetration inhibition ratio (long term) | | | (%) | 90 |
| Ca/P | | | | 1.023 |
| P/L | | | | 0.681 |

Example 51

[Components of polymerizable compositions]

MDP: 10-Methacryloyloxydecyl dihydrogen phosphate

BisGMA: 2,2-Bis[(4-(3-methacryloyloxy)-2-hydroxypropoxyphenyl)]prop ane

HEMA: 2-Hydroxyethylmethacrylate

TMDPO: 2,4,6-Trimethylbenzoyldiphenylphosphine oxide

Inorganic filler 1: R972 produced by Nippon Aerosil Co. , Ltd.

[Preparation of dental adhesive composition]

A one-component self etching type bond was prepared by mixing the following components at normal temperature. One-component bonding material composition :

| | |
|---|---|
| MDP | 10 parts by weight |
| BisGMA | 30 parts by weight |
| HEMA | 30 parts by weight |

(continued)

| TMDPO | 3 parts by weight |
| Water | 15 parts by weight |
| Ethanol | 15 parts by weight |
| Inorganic filler 1 | 5 parts by weight |

[Evaluation of adhesive property]

The labial surface of a bovine mandibular incisor was ground with #80 silicon carbide paper (manufactured by Nihon Kenshi Co., Ltd.) under running water, and thereby a flat surface of dentin was exposed. Subsequently, the sample was further ground with #1000 silicon carbide paper (manufactured by Nihon Kenshi Co., Ltd.) under running water. After completion of the grinding, water on the surface was removed by air blowing and thereby a sample to be adhered was obtained.

A dentinal tubule sealant was rubbed to an area of 4 mm × 4 mm of the dentin surface of the resulting sample to be adhered with a microbrush (Microbrush Superfine produced by Microbrush Corporation) for 30 seconds. Subsequently, the dentin surface was scrubbed with a cotton swab (COTTON PELLETS #3 produced by Richmond) wet with distilled water and thereby a solid component adhering the dentin surface was cleaned off.

An adhesive tape with a thickness of about 150 $\mu$m having a circular hole whose diameter was 3 mm was attached to the sealant-treated surface of the sample to be adhered and thereby the adhesive area was defined. A one-component bonding material composition was applied within the round hole with a brush, followed by being allowed to stand for 20 seconds. Then, the surface was dried by air-blowing until the one-component bonding material composition applied lost its flowability. Then, the resultant was irradiated with light for 20 seconds using a dental visible light irradiator "JET LITE 3000" (manufactured by J. Morita USA, Inc.), thereby curing the one-component bonding material composition applied.

A dental filling composite resin (manufactured by Kuraray Medical Inc., "CLEARFIL AP-X" (trade name, registered trademark)) was applied to the surface of each resultant cured product of the one-component bonding material compositions, and it was then covered with a mold release film (polyester) . Next, slide glass was placed on the mold release film to press it, and thereby the surface of the applied composite resin was smoothed. Subsequently, the composite resin was irradiated with light for 20 seconds using the aforementioned irradiator "JET LITE 3000" through the mold release film. Thus, the composite resin was cured.

To the surface of the resulting cured composite resin for dental filling, one end face (circular section) of a cylindrical bar made of stainless steel (7 mm in diameter and 2.5 cm in length) was adhered with a commercially available dental resin cement (produced by Kuraray Medical Inc., trade name "PANAVIA 21"). After bonding, this sample was allowed to stand still at room temperature for 30 minutes and was then immersed in distilled water. The resultant sample that had been immersed in distilled water was allowed to stand still for 24 hours inside a thermostat whose temperature was maintained at 37°C. Thus, a bonding test sample was produced.

The tensile bond strengths of five bonding test samples were measured with a universal testing machine (manufactured by Shimadzu Corporation), with the crosshead speed being set at 2 mm/min, and the average value thereof was taken as tensile bond strength. Rupture surfaces after the test were observed and the number of samples in which the dentin side was broken was considered as the number of adherent breaks. The tensile adhesion strength in the case of applying a dental adhesive composition to a dentinal tubule surface without using a dentinal tubule sealant was 17.7 (MPa), whereas the tensile adhesion strength in the case of using a dentinal tubule sealant without performing scrubbing for cleaning a solid component adhering to a dentin surface was 8.2 (MPa).

[Table 6]

| | | | Example 51 |
|---|---|---|---|
| Powder agent | DCPA 1.1$\mu$m | (% by weigh) | 55 |
| | Ca(OH)$_2$ 5.2$\mu$m | (% by weigh) | 0.5 |
| Liquid agent | Na$_2$HPO$_4$ | (% by weigh) | 0.5 |
| | NaF | (% by weigh) | 0.22 |
| | Purified water | (% by weigh) | 43.78 |
| Tensile adhesion strength (MPa) | | | 18.1 |
| Number of adherent breaks | | | 4 |

As is shown by the result of the tensile adhesion strength in Example 51, there was obtained adhesion strength comparable to that of the case where a dental adhesive composition was applied to a dentinal tubule surface without using a dentinal tubule sealant. Accordingly, it has become clear that pains, hyperesthesia, and so on are allowed to be suppressed because dentinal tubules will be filled and sealed with solid particles and a dental adhesive composition is allowed to exhibit improved adhesive properties to a dentinal tubule surface because solid components adhering to the dentin surface can be removed by scrubbing using water.

**Claims**

1. A dentinal tubule sealant comprising poorly-soluble calcium phosphate particles (A), a phosphorus-free calcium compound (B), and water (C), wherein
the poorly-soluble calcium phosphate particles (A) are at least one member selected from the group consisting of dicalcium phosphate anhydrous $[CaHPO_4]$ particles, $\alpha$-tricalcium phosphate $[\alpha\text{-}Ca_3(PO_4)_2]$ particles, $\beta$-tricalcium phosphate $[\beta\text{-}Ca_3(PO_4)_2]$ particles, amorphous calcium phosphate $[Ca_3(PO_4)_2 \cdot nH_2O]$ particles, calcium pyrophosphate $[Ca_2P_2O_7]$ particles, calcium pyrophosphate dihydrate $[Ca_2P_2O_7 \cdot 2H_2O]$ particles, octacalcium phosphate pentahydrate $[Ca_8H_2(PO_4)_6 \cdot 5H_2O]$ particles, and dicalcium phosphate dihydrate $[CaHPO_4 \cdot 2H_2O]$ particles, and the dentinal tubule sealant contains 30 to 76% by weight of the poorly-soluble calcium phosphate particles (A), 0.01 to 2% by weight of the phosphorus-free calcium compound (B), and 23 to 69% by weight of the water (C).

2. The dentinal tubule sealant according to claim 1, wherein the phosphorus-free calcium compound (B) is at least one member selected from the group consisting of calcium hydroxide $[Ca(OH)_2]$, calcium oxide $[CaO]$, calcium chloride $[CaCl_2]$, calcium nitrate $[Ca(NO_3)_2 \cdot nH_2O]$, calcium acetate $[Ca(CH_3CO_2)_2 \cdot nH_2O]$, calcium lactate $[C_6H_{10}CaO_6]$, calcium citrate $[Ca_3(C_6H_5O_7)_2 \cdot nH_2O]$, calcium metasilicate $[CaSiO_3]$, dicalcium silicate $[Ca_2SiO_4]$, tricalcium silicate $[Ca_3SiO_5]$, and calcium carbonate $[CaCO_3]$.

3. The dentinal tubule sealant according to claim 1 or 2, further comprising 0.1 to 25% by weight of an alkali metal salt of phosphoric acid (D).

4. The dentinal tubule sealant according to any one of claims 1 to 3, wherein a Ca/P ratio of the poorly-soluble calcium phosphate particle (A) and the phosphorus-free calcium compound (B) in total is from 0.9 to 1.25.

5. The dentinal tubule sealant according to any one of claims 1 to 4, further comprising a fluorine compound (E).

6. The dentinal tubule sealant according to any one of claims 1 to 5, further comprising silica particles (F).

7. The dentinal tubule sealant according to any one of claims 1 to 6 for use in the therapy of hyperesthesia.

8. A tooth surface-treating material comprising the dentinal tubule sealant according to any one of claims 1 to 6.

9. A dentinal hypersensitivity inhibitor comprising the dentinal tubule sealant according to any one of claims 1 to 6.

10. A method for producing a dentinal tubule sealant, the method comprising mixing poorly-soluble calcium phosphate particles (A), a phosphorus-free calcium compound (B), and a liquid or aqueous paste comprising water (C) as a main component, wherein
the poorly-soluble calcium phosphate particles (A) are at least one member selected from the group consisting of dicalcium phosphate anhydrous $[CaHPO_4]$ particles, $\alpha$-tricalcium phosphate $[\alpha\text{-}Ca_3(PO_4)_2]$ particles, $\beta$-tricalcium phosphate $[\beta\text{-}Ca_3(PO_4)_2]$ particles, amorphous calcium phosphate $[Ca_3(PO_4)_2 \cdot nH_2O]$ particles, calcium pyrophosphate $[Ca_2P_2O_7]$ particles, calcium pyrophosphate dihydrate $[Ca_2P_2O_7 \cdot 2H_2O]$ particles, octacalcium phosphate pentahydrate $[Ca_8H_2(PO_4)_6 \cdot 5H_2O]$ particles, and dicalcium phosphate dihydrate $[CaHPO_4 \cdot 2H_2O]$ particles, and the method comprises blending 30 to 76% by weight of the poorly-soluble calcium phosphate particles (A), 0.01 to 2% by weight of the phosphorus-free calcium compound (B), and 23 to 69% by weight of the liquid or aqueous paste comprising water (C) as a main component.

11. The method for producing a dentinal tubule sealant according to claim 10, wherein the method comprises adding the liquid or aqueous paste comprising water (C) as a main component to a powder or nonaqueous paste comprising the poorly-soluble calcium phosphate particles (A) and the phosphorus-free calcium compound (B), and then mixing them.

**12.** The method for producing a dentinal tubule sealant according to claim 10 or 11, wherein a mixing ratio (P/L) is from 0.5 to 3.

**13.** A dentinal tubule sealant kit comprising a powder or nonaqueous paste comprising poorly-soluble calcium phosphate particles (A) and a phosphorus-free calcium compound (B) and a liquid or aqueous paste comprising water (C) as a main component, wherein
the poorly-soluble calcium phosphate particles (A) are at least one member selected from the group consisting of dicalcium phosphate anhydrous $[CaHPO_4]$ particles, $\alpha$-tricalcium phosphate $[\alpha\text{-}Ca_3(PO_4)_2]$ particles, $\beta$-tricalcium phosphate $[\beta\text{-}Ca_3(PO_4)_2]$ particles, amorphous calcium phosphate $[Ca_3(PO_4)_2 \cdot nH_2O]$ particles, calcium pyrophosphate $[Ca_2P_2O_7]$ particles, calcium pyrophosphate dihydrate $[Ca_2P_2O_7 \cdot 2H_2O]$ particles, octacalcium phosphate pentahydrate $[Ca_8H_2(PO_4)_6 \cdot 5H_2O]$ particles, and dicalcium phosphate dihydrate $[CaHPO_4 \cdot 2H_2O]$ particles, and
the kit contains 30 to 76% by weight of the poorly-soluble calcium phosphate particles (A), 0.01 to 2% by weight of the phosphorus-free calcium compound (B), and 23 to 69% by weight of the water (C).

**14.** The dentinal tubule sealant kit according to claim 13, wherein the poorly-soluble calcium phosphate particles (A) have an average particle diameter of 0.8 to 7.5 $\mu$m and the phosphorus-free calcium compound (B) has an average particle diameter of 0.3 to 12 $\mu$m.

**Patentansprüche**

**1.** Dentinkanälchenversiegelungsmittel, umfassend schlechtlösliche Calciumphosphatteilchen (A), eine phosphorfreie Calciumverbindung (B) und Wasser (C), wobei
die schlechtlöslichen Calciumphosphatteilchen (A) mindestens ein Mitglied, ausgewählt aus der Gruppe, bestehend aus wasserfreiem Dicalciumphosphat, $[CaHPO_4]$ Teilchen, $\alpha$-Tricalciumphosphat $[\alpha\text{-}Ca_3(PO_4)_2]$ Teilchen, $\beta$-Tricalciumphosphat $[\beta\text{-}Ca_3(PO_4)_2]$ Teilchen, amorphem Calciumphosphat $[Ca_3(PO_4)_2 \cdot nH_2O]$ Teilchen, Calciumpyrophosphat $[Ca_2P_2O_7]$ Teilchen, Calciumpyrophosphatdihydrat $[Ca_2P_2O_7 \cdot 2H_2O]$ Teilchen, Octacalciumphosphatpentahydrat $[Ca_8H_2(PO_4)_6 \cdot 5H_2O]$ Teilchen und Dicalciumphosphatdihydrat $[CaHPO_4 \cdot 2H_2O]$ Teilchen, sind, und
das Dentinkanälchenversiegelungsmittel 30 bis 76 Gew.-% der schlechtlöslichen Calciumphosphatteilchen (A), 0,01 bis 2 Gew.-% der phosphorfreien Calciumverbindung (B) und 23 bis 69 Gew.-% Wasser (C) enthält.

**2.** Dentinkanälchenversiegelungsmittel gemäß Anspruch 1, wobei die phosphorfreie Calciumverbindung (B) mindestens ein Mitglied, ausgewählt aus der Gruppe, bestehend aus Calciumhydroxid $[Ca(OH)_2]$, Calciumoxid $[CaO]$, Calciumchlorid $[CaCl_2]$, Calciumnitrat $[Ca(NO_3)_2 \cdot nH_2O]$, Calciumacetat $[Ca(CH_3CO_2)_2 \cdot nH_2O]$, Calciumlactat $[C_6H_{10}CaO_6]$, Calciumcitrat $[Ca_3(C_6H_5O_7)_2 \cdot nH_2O]$, Calciummetasilicat $[CaSiO_3]$, Dicalciumsilicat $[Ca_2SiO_4]$, Tricalciumsilicat $[Ca_3SiO_5]$ und Calciumcarbonat $[CaCO_3]$, ist.

**3.** Dentinkanälchenversiegelungsmittel gemäß Anspruch 1 oder 2, weiter umfassend 0,1 bis 25 Gew.-% eines Alkalimetallsalzes von Phosphorsäure (D).

**4.** Dentinkanälchenversiegelungsmittel gemäß einem der Ansprüche 1 bis 3, wobei ein Ca/P Verhältnis des schlechtlöslichen Calciumphosphatteilchens (A) und der phosphorfreien Calciumverbindung (B) im Gesamten von 0,9 bis 1,25 ist.

**5.** Dentinkanälchenversiegelungsmittel gemäß einem der Ansprüche 1 bis 4, weiter umfassend eine Fluorverbindung (E).

**6.** Dentinkanälchenversiegelungsmittel gemäß einem der Ansprüche 1 bis 5, weiter umfassend Silicateilchen (F).

**7.** Dentinkanälchenversiegelungsmittel gemäß einem der Ansprüche 1 bis 6 zur Verwendung in der Therapie von Hyperästhesie.

**8.** Zahnoberflächenbehandelndes Material, umfassend das Dentinkanälchenversiegelungsmittel gemäß einem der Ansprüche 1 bis 6.

**9.** Dentinhypersensibilitätsinhibitor, umfassend das Dentinkanälchenversiegelungsmittel gemäß einem der Ansprüche 1 bis 6.

**10.** Verfahren zur Herstellung eines Dentinkanälchenversiegelungsmittels, wobei das Verfahren das Mischen von schlechtlöslichen Calciumphosphatteilchen (A), einer phosphorfreien Calciumverbindung (B) und einer flüssigen oder wässrigen Paste, umfassend Wasser (C) als eine Hauptkomponente, umfaßt, wobei die schlechtlöslichen Calciumphosphatteilchen (A) mindestens ein Mitglied, ausgewählt aus der Gruppe, bestehend aus wasserfreiem Dicalciumphosphat, $[CaHPO_4]$ Teilchen, $\alpha$-Tricalciumphosphat $[\alpha$-$Ca_3(PO_4)_2]$ Teilchen, $\beta$-Tricalciumphosphat $[\beta$-$Ca_3(PO_4)_2]$ Teilchen, amorphem Calciumphosphat $[Ca_3(PO_4)_2 \cdot nH_2O]$ Teilchen, Calciumpyrophosphat $[Ca_2P_2O_7]$ Teilchen, Calciumpyrophosphatdihydrat $[Ca_2P_2O_7 \cdot 2H_2O]$ Teilchen, Octacalciumphosphatpentahydrat $[Ca_8H_2(PO_4)_6 \cdot 5H_2O]$ Teilchen und Dicalciumphosphatdihydrat $[CaHPO_4 \cdot 2H_2O]$ Teilchen, sind,
und das Verfahren das Mischen von 30 bis 76 Gew.-% der schlechtlöslichen Calciumphosphatteilchen (A), 0,01 bis 2 Gew.-% der phosphorfreien Calciumverbindung (B) und 23 bis 69 Gew.-% der flüssigen oder wässrigen Paste, umfassend Wasser (C) als eine Hauptkomponente, umfaßt.

**11.** Verfahren zur Herstellung eines Dentinkanälchenversieglungsmittels gemäß Anspruch 10, wobei das Verfahren das Zugeben der flüssigen oder wässrigen Paste, umfassend Wasser (C) als eine Hauptkomponente, zu einem Pulver oder einer nicht-wässrigen Paste, umfassend die schlechtlöslichen Calciumphosphatteilchen (A) und die phosphorfreie Calciumverbindung (B), und anschließend Mischen dieser umfaßt.

**12.** Verfahren zur Herstellung eines Dentinkanälchenversiegelungsmittels gemäß Anspruch 10 oder 11, wobei ein Mischungsverhältnis (P/L) von 0,5 bis 3 beträgt.

**13.** Dentinkanälchenversiegelungskit, umfassend ein Pulver oder eine nichtwässrige Paste, umfassend schlechtlösliche Calciiumphosphatteilchen (A) und eine phosphorfreie Calciumverbindung (B), und eine flüssige oder wässrige Paste, umfassend Wasser (C) als eine Hauptkomponente, wobei
die schlechtlöslichen Calciumphosphatteilchen (A) mindestens ein Mitglied, ausgewählt aus der Gruppe, bestehend aus wasserfreiem Dicalciumphosphat, $[CaHPO_4]$ Teilchen, $\alpha$-Tricalciumphosphat $[\alpha$-$Ca_3(PO_4)_2]$ Teilchen, $\beta$-Tricalciumphosphat $[\beta$-$Ca_3(PO_4)_2]$ Teilchen, amorphem Calciumphosphat $[Ca_3(PO_4)_2 \cdot nH_2O]$ Teilchen, Calciumpyrophosphat $[Ca_2P_2O_7]$ Teilchen, Calciumpyrophosphatdihydrat $[Ca_2P_2O_7 \cdot 2H_2O]$ Teilchen, Octacalciumphosphatpentahydrat $[Ca_8H_2(PO_4)_6 \cdot 5H_2O]$ Teilchen und Dicalciumphosphatdihydrat $[CaHPO_4 \cdot 2H_2O]$ Teilchen, sind,
und das Kit 30 bis 76 Gew.-% der schlechtlöslichen Calciumphosphatteilchen (A), 0,01 bis 2 Gew.-% der phosphorfreien Calciumverbindung (B) und 23 bis 69 Gew.-% von dem Wasser (C) enthält.

**14.** Dentinkanälchenversiegelungskit gemäß Anspruch 13, wobei die schlechtlöslichen Calciumphosphatteilchen (A) einen durchschnittlichen Teilchendurchmesser von 0,8 bis 7,5 $\mu$m aufweisen und die phosphorfreie Calciumverbindung (B) einen durchschnittlichen Teilchendurchmesser von 0,3 bis 12 $\mu$m aufweist.

**Revendications**

**1.** Agent obturant de tubules dentinaires, comprenant des particules de phosphate de calcium peu solubles (A), un composé du calcium sans phosphore (B) et de l'eau (C), où
les particules de phosphate de calcium peu solubles (A) sont au moins un membre choisi parmi le groupe consistant en des particules de phosphate dicalcique anhydre $[CaHPO_4]$, des particules de phosphate tricalcique $\alpha$ $[\alpha$-$Ca_3(PO_4)_2]$, des particules de phosphate tricalcique $\beta[\beta$-$Ca_3(PO_4)_2]$, des particules de phosphate de calcium amorphe $[Ca_3(PO_4)_2 \cdot nH_2O]$, des particules de pyrophosphate de calcium $[Ca_2P_2O_7]$, des particules de pyrophosphate de calcium dihydraté $[Ca_2P_2O_7 \cdot 2H_2O]$, des particules de phosphate d'octacalcium pentahydraté $[Ca_8H_2(PO_4)_6 \cdot 5H_2O]$, et des particules de phosphate dicalcique dihydraté $[CaHPO_4 \cdot 2H_2O]$, et
l'agent obturant de tubules dentinaires contient 30 à 76% en poids de particules de phosphate de calcium peu solubles (A), 0,01 à 2% en poids de composé du calcium sans phosphore (B), et 23 à 69% en poids d'eau (C).

**2.** Agent obturant de tubules dentinaires selon la revendication 1, où le composé du calcium sans phosphore (B) est au moins un membre choisi parmi le groupe consistant en l'hydroxyde de calcium $[Ca(OH)_2]$, l'oxyde de calcium $[CaO]$, le chlorure de calcium $[CaCl_2]$, le nitrate de calcium $[Ca(NO_3)_2 \cdot nH_2O]$, l'acétate de calcium $[Ca(CH_3CO_2)_2 \cdot nH_2O]$, le lactate de calcium $[C_6H_{10}CaO_6]$, le citrate de calcium $[Ca_3(C_6H_5O_7)_2 \cdot nH_2O]$, le métasilicate de calcium $[CaSiO_3]$, le silicate de dicalcium $[Ca_2SiO_4]$, le silicate de tricalcium $[Ca_3SiO_5]$ et le carbonate de calcium $[CaCO_3]$.

**3.** Agent obturant de tubules dentinaires selon la revendication 1 ou 2, comprenant en outre, 0,1 à 25% en poids d'un sel de métal alcalin de l'acide phosphorique (D).

**4.** Agent obturant de tubules dentinaires selon l'une quelconque des revendications 1 à 3, où le rapport Ca/P des particules de phosphate de calcium peu solubles (A) et du composé du calcium sans phosphore se situe dans l'intervalle allant de 0,9 à 1,25.

**5.** Agent obturant de tubules dentinaires selon l'une quelconque des revendications 1 à 4, comprenant en outre, un composé du fluor (E).

**6.** Agent obturant de tubules dentinaires selon l'une quelconque des revendications 1 à 5, comprenant en outre, des particules de silice (F).

**7.** Agent obturant de tubules dentinaires selon l'une quelconque des revendications 1 à 6, à utiliser dans le traitement de l'hyperesthésie.

**8.** Matériau de traitement de surface des dents comprenant l'agent obturant de tubules dentinaires selon l'une quelconque des revendications 1 à 6.

**9.** Inhibiteur de l'hypersensibilité dentaire comprenant l'agent obturant de tubules dentinaires selon l'une quelconque des revendications 1 à 6.

**10.** Procédé de préparation d'un agent d'obturation de tubules dentinaires, le procédé comprenant le mélange de particules de phosphate de calcium peu solubles (A), d'un composé du calcium sans phosphate (B) et d'un liquide ou d'une pâte aqueuse comprenant de l'eau (C) comme composant principaux, où
les particules de phosphate de calcium peu solubles (A) sont au moins un membre choisi parmi le groupe consistant en des particules de phosphate dicalcique anhydre [$CaHPO_4$], des particules de phosphate tricalcique $\alpha$ [$\alpha$-$Ca_3(PO_4)_2$], des particules de phosphate tricalcique $\beta$[$\beta$-$Ca_3(PO_4)_2$], des particules de phosphate de calcium amorphe [$Ca_3(PO_4)_2.nH_2O$], des particules de pyrophosphate de calcium [$Ca_2P_2O_7$], des particules de pyrophosphate de calcium dihydraté [$Ca_2P_2O_7.2H_2O$], des particules de phosphate d'octacalcium pentahydraté [$Ca_8H_2(PO_4)_6.5H_2O$], et des particules de phosphate dicalcique dihydraté [$CaHPO_4.2H_2O$], et
le procédé comprend le mélange de 30 à 76% en poids de particules de phosphate de calcium peu solubles (A), 0,01 à 2% en poids de composé du calcium sans phosphore (B) et 23 à 69% en poids du liquide ou de la pâte aqueuse comprenant de l'eau (C) comme composants principaux.

**11.** Procédé de préparation d'un agent d'obturation de tubules dentinaires selon la revendication 10, où le procédé comprend l'addition du liquide ou de la pâte aqueuse comprenant de l'eau (C) comme composant principal à une poudre ou pâte non aqueuse comprenant les particules de phosphate de calcium peu solubles (A) et le composé du calcium sans phosphore (B), puis leur mélange.

**12.** Procédé de préparation d'un agent d'obturation de tubules dentinaires selon la revendication 10 ou 11, où le rapport de mélange (P/L) se situe dans l'intervalle allant de 0,5 à 3.

**13.** Kit d'obturation de tubules dentinaires comprenant une poudre ou une pâte non aqueuse comprenant les particules de phosphate de calcium peu solubles (A) et un composé du calcium sans phosphore (B) et un liquide ou une pâte aqueuse comprenant de l'eau (C) comme composants principaux, où
les particules de phosphate de calcium peu solubles (A) sont au moins un membre choisi parmi le groupe consistant en des particules de phosphate dicalcique anhydre [$CaHPO_4$], des particules de phosphate tricalcique $\alpha$ [$\alpha$-$Ca_3(PO_4)_2$], des particules de phosphate tricalcique $\beta$[$\beta$-$Ca_3(PO_4)_2$], des particules de phosphate de calcium amorphe [$Ca_3(PO_4)_2.nH_2O$], des particules de pyrophosphate de calcium [$Ca_2P_2O_7$], des particules de pyrophosphate de calcium dihydraté [$Ca_2P_2O_7.2H_2O$], des particules de phosphate d'octacalcium pentahydraté [$Ca_8H_2(PO_4)_6.5H_2O$], et des particules de phosphate dicalcique dihydraté [$CaHPO_4.2H_2O$], et
le kit contient 30 à 76% en poids de particules de phosphate de calcium peu solubles (A), 0,01 à 2% en poids de composé du calcium sans phosphore (B), et 23 à 69% en poids d'eau (C).

**14.** Kit d'obturation de tubules dentinaires selon la revendication 13, où les particules de phosphate de calcium peu solubles (A) ont un diamètre particulaire moyen situé dans l'intervalle allant de 0,8 à 7,5 $\mu$m et le composé du calcium sans phosphore (B) a un diamètre moyen des particules allant de 0,3 à 12 $\mu$m.

Figure 1

Figure 2
(a)                                                              (b)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6172008 A
- JP 10504467 A
- JP 2000504037 A
- US 6053970 A

**Non-patent literature cited in the description**

- **D. H.PASHLEY et al.** *J. Dent. Res.,* 1986, vol. 65, 417-420
- **K. C. Y. TAY et al.** *J. Endod.,* 2007, vol. 33, 1438-1443